# EUROPEAN PATENT SPECIFICATION

(11) **EP 1 748 994 B1**
(45) Date of publication and mention of the grant of the patent: **18.02.2009**
(21) Application number: 04803433.4
(22) Date of filing: 01.12.2004
(51) Int. Cl.: C07D 285/10, A61K 31/433, A61P 9/00

(54) **NITROOXYDERIVATIVES OF ANTIHYPERTENSIVE DRUGS**
NITROOXYDERIVATE VON BLUTDRUCKSENKENDEN ARZNEIMITTELN
DERIVES DE NITROOXY DES MEDICAMENTS ANTIHYPERTENSEURS

(30) Priority: 02.12.2003 EP 03104485
(43) Date of publication of application: 07.02.2007
(73) Proprietor: NicOx S.A., 06560 Sophia Antipolis - Valbonne (FR)
(72) Inventor: DEL SOLDATO, Piero, I-20052 Monza (IT); BENEDINI, Francesca, I-20132 Milano (IT); ONGINI, Ennio, I-20090 Segrate (IT)
(74) Representative: Minoja, Fabrizio
(86) International application number: PCT/EP2004/013682
(87) International publication number: WO 2005/054218

(56) References cited:
- WO-A-00/61541
- WO-A-01/12584
- US-A- 4 801 596
- US-A- 5 639 904

## Description

The present invention relates to β-adrenergic blockers derivatives. More particularly, the present invention relates to β-adrenergic blockers nitrooxyderivatives, pharmaceutical compositions containing them and their use for the treatment of hypertension, cardiovascular diseases, glaucoma, migraine headache, vascular diseases and elevated intraocular pressure.

β-adrenergic blockers (β-blockers) are widely used in the treatment of hypertension and cardiovascular diseases including angina pectoris, arrhythmias, acute myocardial infarction, hypertrophic cardiomyopathy, congestive heart failure.
They work to block the effects of catecholamines at receptor sites in the heart, but they differ somewhat in their ability to block receptors in the blood vessels and lungs. Selective β-blockers have their major actions on the heart, some others are weak stimulators of the β-preceptor while still blocking the major actions of catecholamines, some block both the β₁ and β₂ receptors in the heart and those in the blood vessels and have no stimulatory activity and some block other cathecolamine receptors that can lead to further vascular effects on blood vessels.

Several side effects are associated with this class of drugs such as muscle fatigue, sleep disturbances, decreased heart rate, hypotension, cold extremities, bronchospasm in asthmatic patients, hypoglycaemia, increased lipids in plasma.
Moreover, abrupt withdrawal after long-term treatment with beta-blockers has to be avoided, because an increased sensitivity to β-adrenergic system develops.

U.S. Pat. No. 6,242,432 discloses derivatives of formula A-(X₁-NO₂)ₜₒ having an antithrombotic activity, wherein A is the residue of a β-adrenergic blocker, X₁ is a bivalent connecting bridge and tₒ is 1 or 2. The invention is limited to particular meanings of the bivalent connecting bridge X₁.

U.S. Pat . No 5,502,237 and U.S. Pat. No 5,639,904 disclose derivatives of formula R₁-Ar-O-CH₂-CH(OH)-CH₂-NH-CH(CH₃)₂ used for the treatment of cardiovascular affections, wherein R₁ is a chain having at least one nitrooxy group as substituent.

U.S. Pat. No. 4,801,596 discloses aminopropanol derivatives of formula 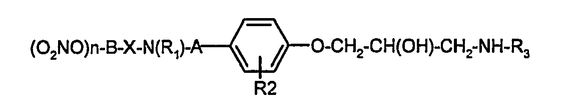 that can be used for prophylaxis and/or treatment of heart and circulatory diseases, wherein R₃ is an alkyl or a nitroxyalkyl radical containing 3 to 8 carbon atoms.

It was an object of the present invention to provide new β-adrenergic blockers nitroxyderivatives having a significantly improved overall pharmacological profile as compared to native β-blockers that are able not only to eliminate or at least reduce the side effects associated with their parent compounds, but also having an improved pharmacological activity and tolerability.

It has been so surprisingly found that the β-adrenergic blockers nitrooxyderivatives of the present invention have a better pharmacological activity and organ protection properties, enhanced effects as anti-inflammatory, and on renal functions. In addition, they are effective in other pathologies including atherosclerosis, diabetes, peripheral vascular diseases (PVD) and elevated intraocular pressure.

In particular, it has been recognized that the β-adrenergic blockers nitrooxyderivatives of the present invention, differently from the above mentioned compounds of the prior art, exhibit an improved activity on the cardiovascular system and enhanced tolerability and can be employed for treating or preventing hypertension, cardiovascular diseases, glaucoma, migraine headache, vascular diseases and elevated intraocular pressure..

Object of the present invention are β-adrenergic blockers nitrooxyderivatives of general formula (I):

A-(Y-ONO₂)ₛ

and enantiomers and diastereoisomers and pharmaceutically acceptable salts thereof, wherein s is an integer equal to 1 or 2, preferably s is 2;
A is selected from the following β-adrenergic blocker residues of formula (II): 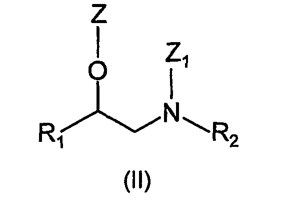 wherein
R₁ is selected from the group consisting of: 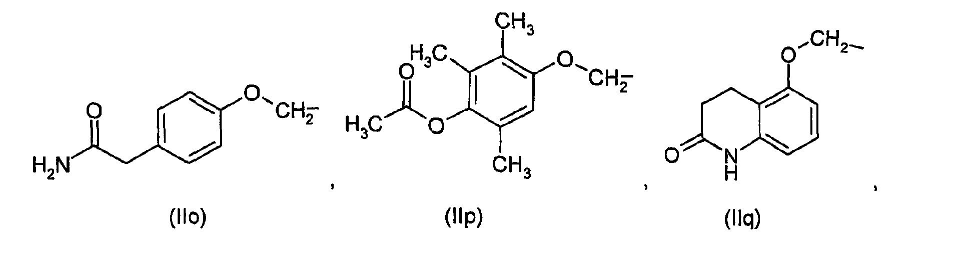 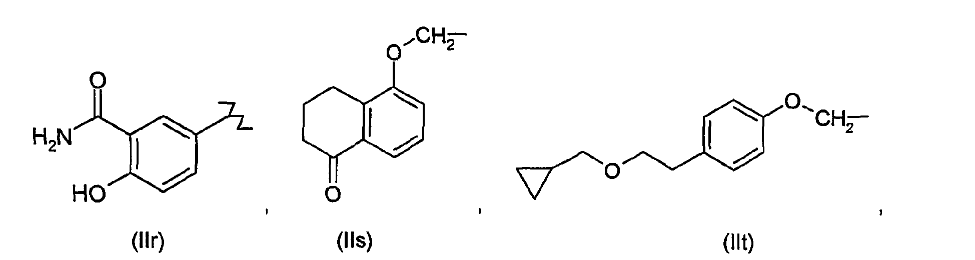 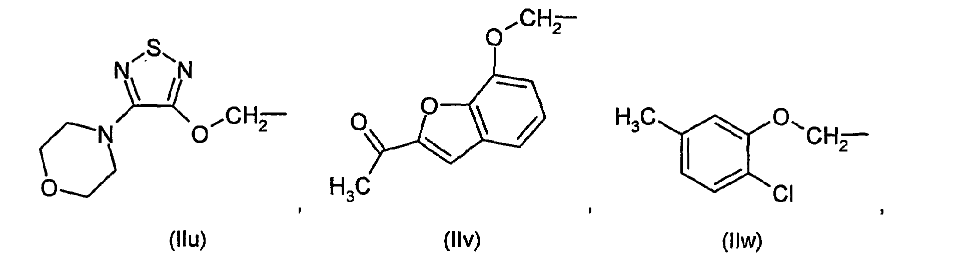 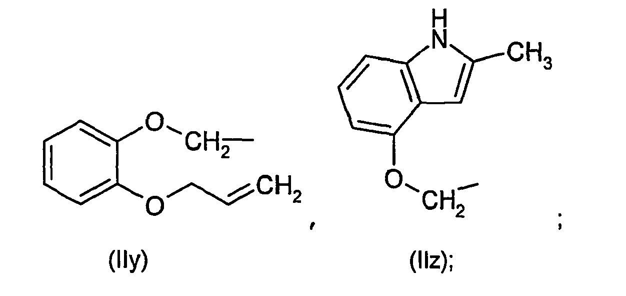 R₂ is selected from the group consisting of: -CH(CH₃)₂, -C(CH₃)₃ or 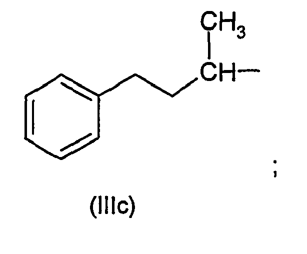 when the radical R₁ has chosen from the formulae (IIo), (IIp), (IIt), (IIu), (IIv), (IIy) or (IIz),
R₂ is -CH(CH₃)₂;
when the radical R₁ has chosen from the formulae (IIq), (IIs) or (IIw), R₂ is -C(CH₃)₃;
when the radical R₁ is (IIr), R₂ is (IIIc);
Z is H or is a group capable of binding Y selected from the group consisting of:
-C(O)-, -C(O)O- or 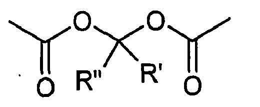 wherein R' and R" are the same or different, and are H or straight or branched C₁-C₄ alkyl;
Z₁ is H or a -C(O)- group capable of binding Y;
with the proviso that when s of formula (I) is 1 Z or Z₁ is H;
when s is 2, Z and Z₁ are preferably -C(O)-;
Y is a bivalent radical having the following meaning:
a)
   - straight or branched C₁-C₂₀ alkylene, preferably C₁-C₁₀, being optionally substituted with one or more of the substituents selected from the group consisting of: halogen atoms, hydroxy, -ONO₂ or T, wherein T is -OC(O)(C₁-C₁₀alkyl)-ONO₂, -O(C₁-C₁₀alkyl)-ONO₂;
b)
   - cycloalkylene with 5 to 7 carbon atoms into cycloalkylene ring, the ring being optionally substituted with side chains T₁, wherein T₁ is straight or branched alkyl with from 1 to 10 carbon atoms, T₁ is preferably CH₃;
c) 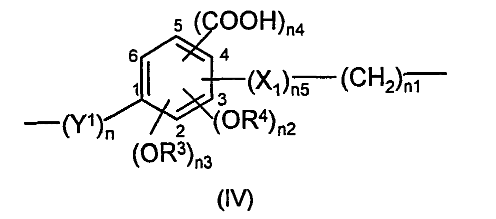 wherein:
   n is an integer from 0 to 20, preferably n is an integer from 0 to 10, more preferably n is 0,
   and n1 is an integer from 1 to 20, preferably from 1 to 10;
   n2, n3, n4 and n5 are integers equal or different from each others, equal to 0 or 1;
   R³ and R⁴ are independently selected from H or CH₃;
   Y¹ is -CH₂- or -(CH₂)ₙₐ-CH=CH- wherein na is an integer from 0 to 20, preferably na is equal to 0;
   X₁ is WC(O)- or-C(O)W-, wherein W is oxygen, sulfur or NH, preferably W is oxygen;
   with the proviso that:
      - when s of formula (I) is 1, Z is -(CO)- and in formula (IV) of the bivalent radical Y n2, n3, n4, n5 are equal to 0 then n is 0 and n1 is 1;
      - when s of formula (I) is 1, Z is -(CO)- and in formula (IV) of the bivalent radical Y n2, n3, n5 are equal to 0, n4 is 1 then n and n1 are different to 1;
d) 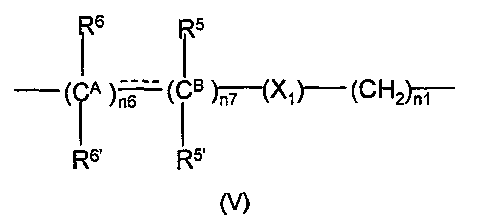 wherein:
   n1 is an integer from 1 to 20, preferably from 1 to 10;
   X₁ is -WC(O)- or -C(O)W-, wherein W is oxygen, sulfur or NH, preferably W is sulfur;
   n6 is an integer from 1 to 20,
   n7 is an integer from 0 to 20,
   R⁵ and R^{5'} R⁶ and R^{6'} are independently selected from the group consisting of: H, CH₃, OH, NH₂, NHCOCH₃, COOH, CH₂SH and C(CH₃)₂SH; when the bond between the C^{A} and C^{B} carbons is a double bond R⁵ and R⁶ or R⁶' and R^{5'} are absent;
   with the proviso that when Y is selected from the bivalent radicals mentioned under c)-d), the -ONO₂ group is linked to a -(CH₂)ₙ₁- group;
   with the proviso that when s of formula (I) is 1 and Z is -(CO)- then the bivalent radical Y has not the meanings under a), b) and d);
e) 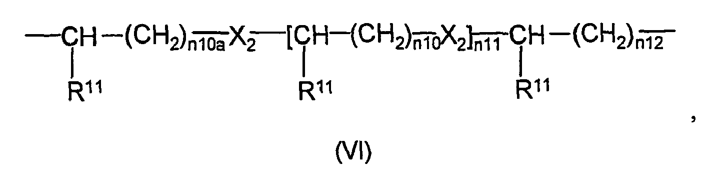 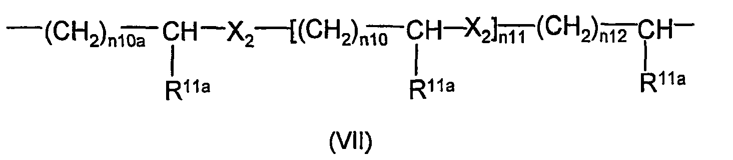 wherein X₂ is O or S,
   n10a, n10 and n12 are integer independently selected from 0 to 20,
   n10a is preferably selected from 0 to 10,
   n10 and n12 are preferably selected from 1 to 10, and
   n11 is an integer from 0 to 6, preferably from 0 to 4,
   R¹¹ is H, CH₃ or nitrooxy group, preferably R¹¹ is H,
   R^{11a} is CH₃ or nitrooxy group;
   with the proviso that when in formula (I) s is 1, in formula (II) Z is -(CO)-, in formula (VI) of the bivalent radical Y n10a, n10, n12 are equal to 1 then X can not be an oxygen atom;
f) 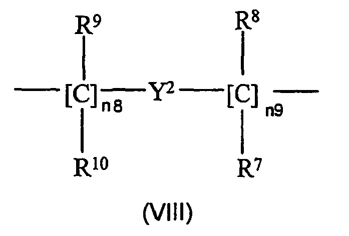 wherein
   n8 is an integer from 0 to 10;
   n9 is an integer from 1 to 10;
   R⁹, R¹⁰, R⁸, R⁷ are same or different, and are H or straight or branched C₁-C₄ alkyl, preferably R⁹, R¹⁰, R⁸, R⁷ are H;
   wherein the -ONO₂ group is linked to 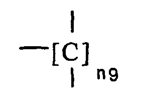 wherein n9 is as defined above;
   Y² is an heterocyclic saturated, unsaturated or aromatic 5 or 6 members ring, containing one or more heteroatom/s selected from nitrogen, oxygen, sulfur,
   and is selected from the group consisting of

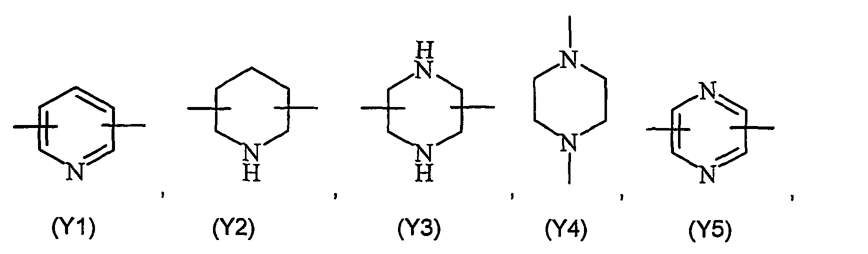

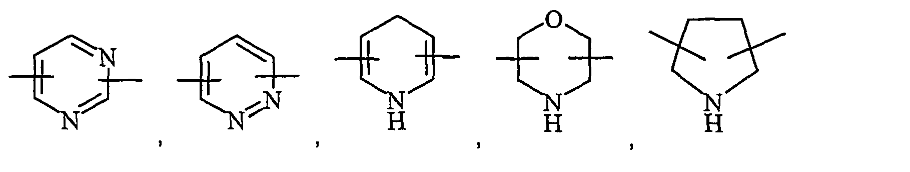

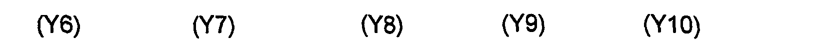

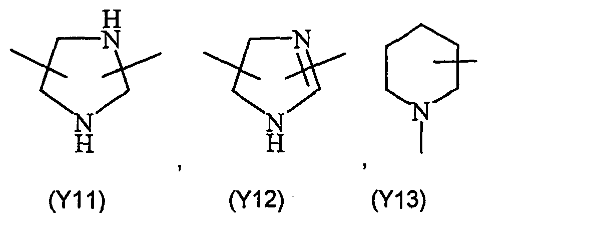

One embodiment of the present invention comprises compounds of formula (I) wherein s is 2,
A is a β-adrenergic blocker residue of formula (II) as above defined:
Z is a group capable of binding Y selected from the group consisting of:
-C(O)-, -C(O)O- or 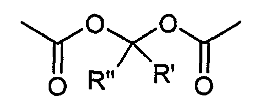 wherein R' and R" are the same or different, and are H or straight or branched C₁-C₄ alkyl;
Z₁ is -C(O)-;
preferably Z and Z₁ are -C(O)-;
Y is a bivalent radical having the following meaning:
a)
   - straight or branched C₁-C₂₀ alkylene, preferably C₁-C₁₀, being optionally substituted with one or more of the substituents selected from the group consisting of: halogen atoms, hydroxy, -ONO₂ or T, wherein T is -OC(O)(C₂-C₁₀alkyl)-ONO₂, -O(C₁-C₁₀)alkyl)-ONO₂;
b)
   - cycloalkylene with 5 to 7 carbon atoms into cycloalkylene ring, the ring being optionally substituted with side chains T₁, wherein T₁ is straight or branched alkyl with from 1 to 10 carbon atoms, T₁ is preferably CH₃;
c) 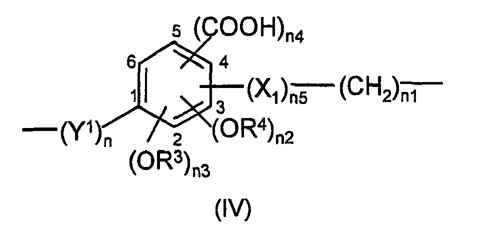 wherein:
   n is an integer from 0 to 20, preferably n is an integer from 0 to 10, more preferably n is 0, and n1 is an integer from 1 to 20, preferably from 1 to 10;
   n2, n3, n4 and n5 are integers equal or different from each others, equal to 0 or 1;
   R³ and R⁴ are independently selected from H or CH₃;
   Y¹ is -CH₂- or -(CH₂)ₙₐ-CH=CH- wherein na is an integer from 0 to 20, preferably na is equal to 0;
   X₁ is WC(O)- or -C(O)W-, wherein W is oxygen, sulfur or NH, preferably W is oxygen;
d) 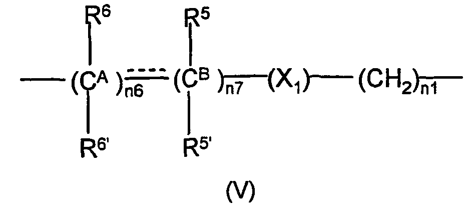 wherein:
   n1 is an integer from 1 to 20, preferably from 1 to 10;
   X₁ is WC(O)- or -C(O)W-, wherein W is oxygen, sulfur or NH, preferably W is sulphur;
   n6 is an integer from 1 to 20, preferably n6 is 1,
   n7 is an integer from 0 to 20, preferably n7 is 1,
   R⁵ and R^{5'} R⁶ and R^{6'}are independently selected from the group consisting of: H, CH₃, OH, NH₂, NHCOCH₃, COOH, CH₂SH and C(CH₃)₂SH; when the bond between the C^{A} and C^{B} carbons is a double bond R⁵ and R⁶ or R⁶' and R^{5'} are absent;
   with the proviso that when Y is selected from the bivalent radicals mentioned under c)-d), the -ONO₂ group is linked to a -(CH₂)ₙ₁- group;
e) 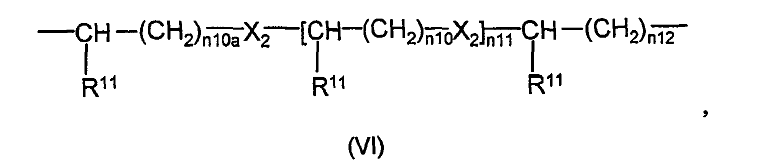 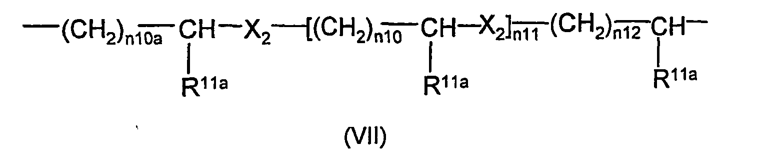 wherein X₂ is O or S,
   n10a, n10 and n12 are integer independently selected from 0 to 20,
   n10a is preferably selected from 0 to 10,
   n10 and n12 are preferably selected from 1 to 10, and
   n11 is an integer from 0 to 6, preferably from 0 to 4,
   R¹¹ is H, CH₃ or nitrooxy group, preferably R¹¹ is H,
   R^{11a} is CH₃ or nitrooxy group;
f) 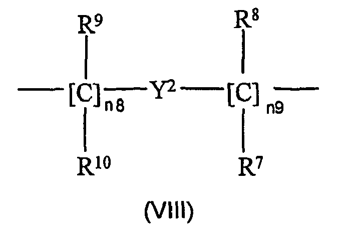 wherein
   n8 is an integer from 0 to 10;
   n9 is an integer from 1 to 10;
   R⁹, R¹⁰, R⁸, R⁷ are same or different, and are H or straight or branched C₁-C₄ alkyl, preferably R⁹, R¹⁰, R⁸, R⁷are H;
   wherein the -ONO₂ group is linked to 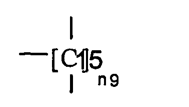 wherein n9 is as defined above;
   Y² is an heterocyclic saturated, unsaturated or aromatic 5 or 6 members ring, containing one or more heteroatom/s selected from nitrogen, oxygen, sulfur,
   and is selected from the group consisting of

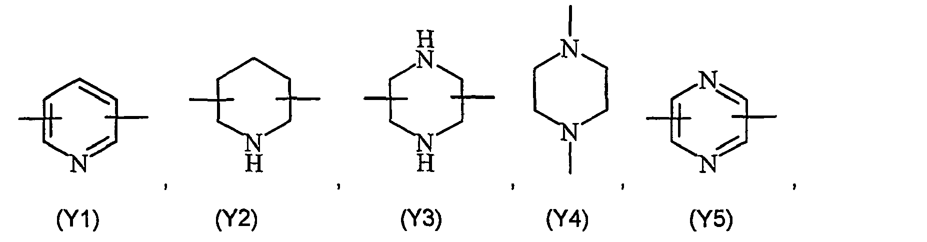

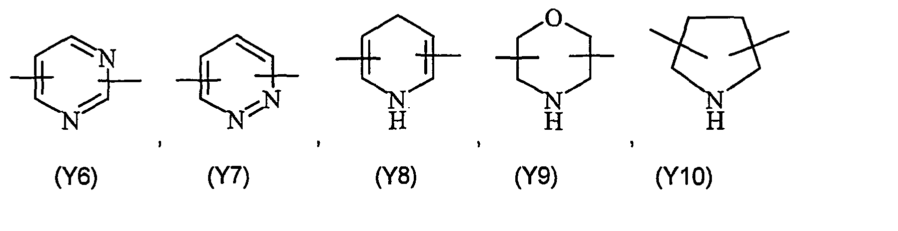

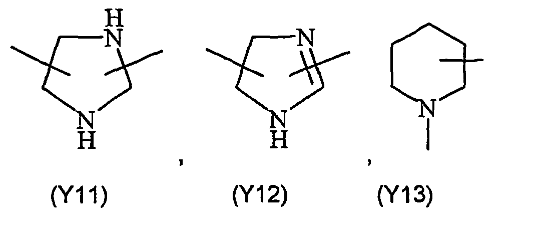

Another embodiment comprises compounds of formula (I) wherein
sis1,
A is a β-adrenergic blocker residue of formula (II) as above defined:
Z is H,
Z₁ is -C(O)-;
Y is a bivalent radical having the following meaning:
a)
   - straight or branched C₁-C₂₀ alkylene, preferably C₁-C₁₀, more preferably C₃-C₆ being optionally substituted with one or more of the substituents selected from the group consisting of: halogen atoms, hydroxy, -ONO₂ or T, wherein T is -OC(O)(C₁-C₁₀alkyl)-ONO₂, -O(C₁-C₁₀alkyl)-ONO₂;
b)
   - cycloalkylene with 5 to 7 carbon atoms into cycloalkylene ring, the ring being optionally substituted with side chains T₁, wherein T₁ is straight or branched alkyl with from 1 to 10 carbon atoms, T₁ is preferably CH₃;
c) 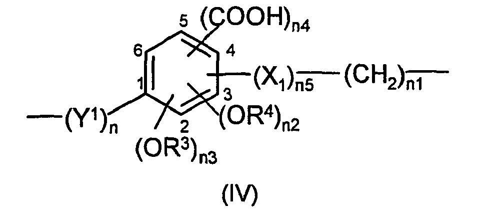 wherein:
   n is an integer from 0 to 20, preferably n is an integer from 0 to 10, more preferably n is 0, and n1 is an integer from 1 to 20, preferably from 1 to 10;
   n2, n3, n4 and n5 are integers equal or different from each others, equal to 0 or 1;
   R³ and R⁴ are independently selected from H or CH₃;
   Y¹ is -CH₂- or -(CH₂)ₙₐ-CH=CH- wherein na is an integer from 0 to 20, preferably na is equal to 0;
   X₁ is WC(O)- or -C(O)W-, wherein W is oxygen, sulfur or NH, preferably W is oxygen;
d) 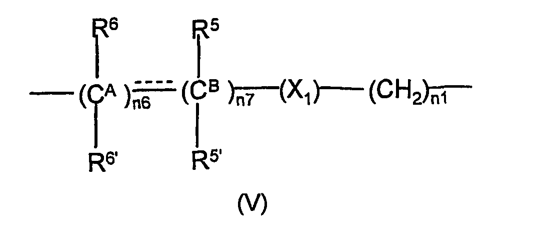 wherein:
   n1 is an integer from 1 to 20, preferably from 1 to 10;
   X₁ is WC(O)- or-C(O)W-, wherein W is oxygen, sulfur or NH, preferably W is sulfur;
   n6 is an integer from 1 to 20,
   n7 is an integer from 0 to 20,
   R⁵ and R^{5'} R⁶ and R^{6'} are independently selected from the group consisting of: H, CH₃, OH, NH₂, NHCOCH₃, COOH, CH₂SH and C(CH₃)₂SH; when the bond between the C^{A} and C^{B} carbons is a double bond R⁵ and R⁶ or R^{6'} and R^{5'} are absent;
   with the proviso that when Y is selected from the bivalent radicals mentioned under c)-d), the -CNO₂ group is linked to a -(CH₂)ₙ₁- group;
e) 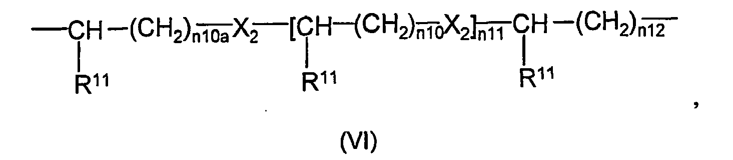 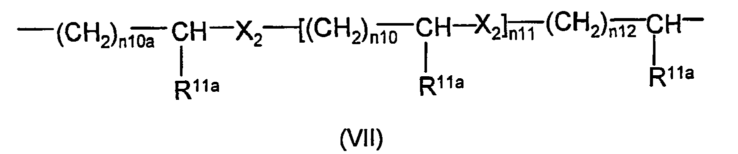 wherein X₂ is O or S,
   n10a, n10 and n12 are integer independently selected from 0 to 20,
   n10a is preferably selected from 0 to 10,
   n10 and n12 are preferably selected from 1 to 10, and
   n11 is an integer from 0 to 6, preferably from 0 to 4,
   R¹¹ is H, CH₃ or nitrooxy group, preferably R¹¹ is H,
   R^{11a} is CH₃ or nitrooxy group;
f) 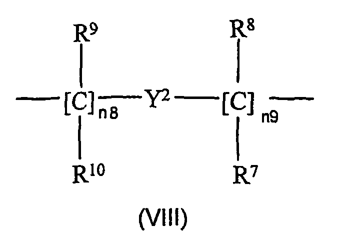 wherein
   n8 is an integer from 0 to 10;
   n9 is an integer from 1 to 10;
   R⁹, R¹⁰, R⁸, R⁷ are same or different, and are H or straight or branched C₁-C₄ alkyl, preferably R⁹, R¹⁰, R⁸, R⁷are H;
   wherein the -ONO₂ group is linked to 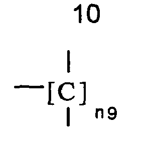 wherein n9 is as defined above;
   Y² is an heterocyclic saturated, unsaturated or aromatic 5 or 6 members ring, containing one or more heteroatom/s selected from nitrogen, oxygen, sulfur,
   and is selected from the group consisting of 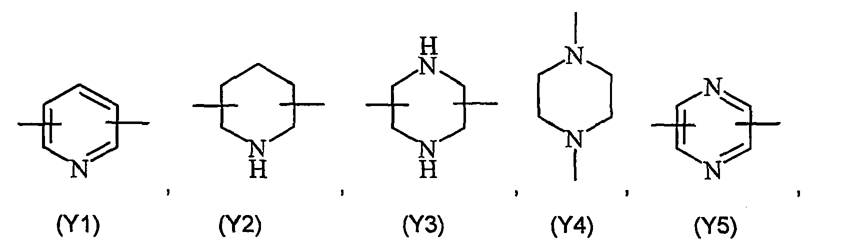 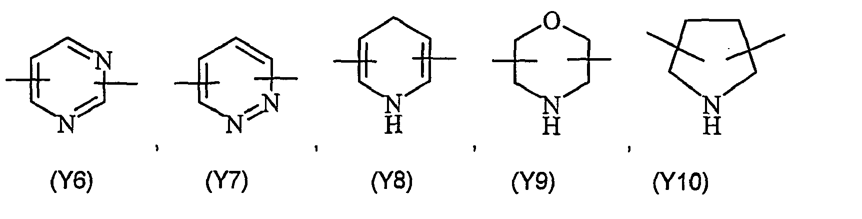 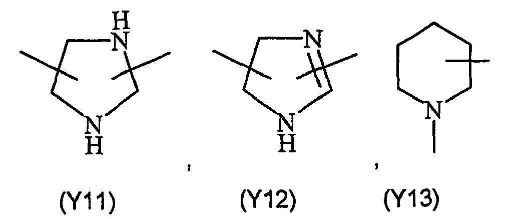
   Another embodiment comprises compounds of formula (I) wherein
   sis1,
   A is a β-adrenergic blocker residue of formula (II) as above defined:
   Z₁ is H,
   Z is a group capable of binding Y selected from the group consisting of: -C(O)-, -C(O)O- or 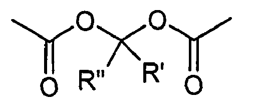 wherein R' and R" are the same or different, and are H or straight or branched C₁-C₄alkyl; preferably Z is -C(O)-;
   Y is a bivalent radical having the following meaning:
c) 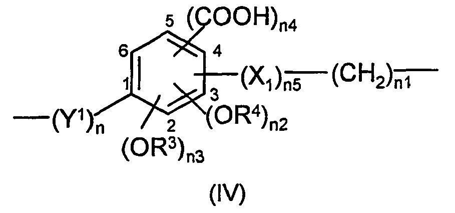 wherein:
   n is an integer from 0 to 20, preferably n is an integer from 0 to 10, more preferably n is 0, and n1 is an integer from 1 to 20, preferably from 1 to 10;
   n2, n3, n4 and n5 are integers equal or different from each others, equal to 0 or 1;
   R³ and R⁴ are independently selected from H or CH₃;
   Y¹ is -CH₂- or -(CH₂)ₙₐ-CH=CH- wherein na is an integer from 0 to 20, preferably na is equal to 0;
   X₁ is WC(O)- or-C(O)W-, wherein W is oxygen, sulfur or NH, preferably W is oxygen; with the proviso that when Z is -C(O)-:
      - in the bivalent radical Y of formula (IV) n2, n3, n4, n5 are equal to 0 then n is 0 and n1 is 1;
      - in the bivalent radical Y of formula (IV) n2, n3, n5 are equal to 0, n4 is 1 then n and n1 are different to 1;
e) 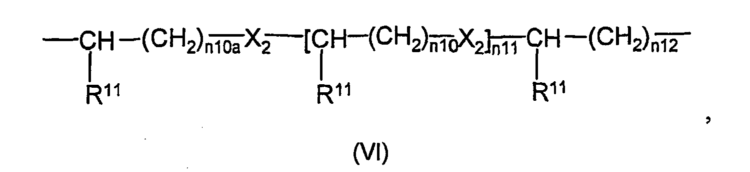 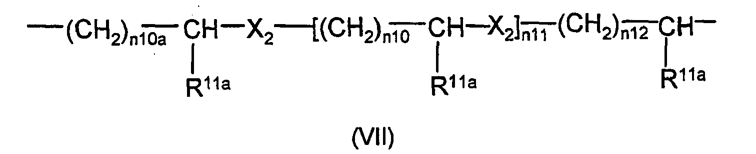 wherein X₂ is O or S,
   n10a, n10 and n12 are integer independently selected from 0 to 20,
   n10a is preferably selected from 0 to 10,
   n10 and n12 are preferably selected from 1 to 10, and
   n11 is an integer from 0 to 6, preferably from 0 to 4,
   R¹¹ is H, CH₃ or nitrooxy group, preferably R¹¹ is H,
   R^{11a} is CH₃ or nitrooxy group;
   with the proviso that when Z is -C(O)- and in formula (VI) of the bivalent radical Y n10a, n10, n12 are equal to 1 then X can not be an oxygen atom;
f) 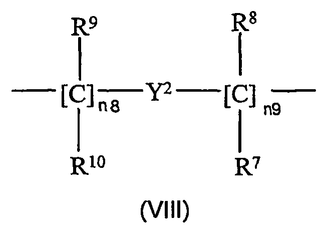 wherein
   n8 is an integer from 0 to 10;
   n9 is an integer from 1 to 10;
   R⁹, R¹⁰, R⁸, R⁷ are same or different, and are H or straight or branched C₁-C₄ alkyl, preferably R⁹, R¹⁰, R⁸, R⁷ are H;
   wherein the -ONO₂ group is linked to 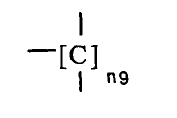 wherein n9 is as defined above;
   Y² is an heterocyclic saturated, unsaturated or aromatic 5 or 6 members ring, containing one or more heteroatom/s selected from nitrogen, oxygen, sulfur,
   and is selected from the group consisting of

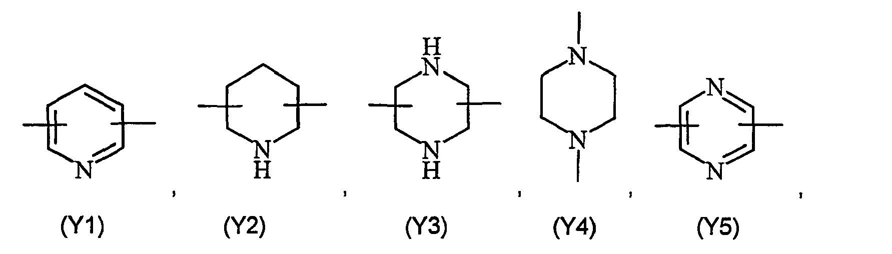

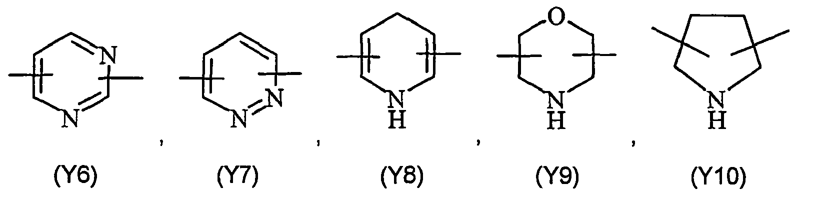

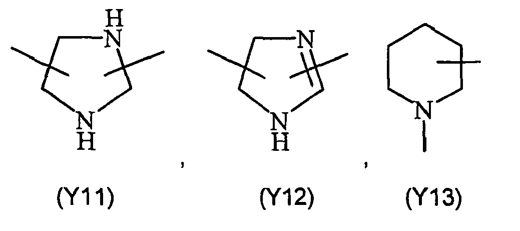

Preferred compounds are those of formula (I) wherein
s is 1
A is a β-adrenergic blocker residue of formula (II) as above defined,
Z is H and Z₁ is -C(O)-,
and the bivalent radical Y have the following meaning:
a) straight C₁-C₁₀ alkylene, preferably C₃-C₆ alkylene;
c) 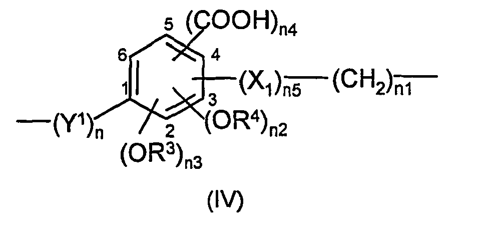 wherein the -ONO₂ group is bound to (CH₂)ₙ₁;
   n, n2, n3, n4, n5 are equal to 0,
   n1 is 1 and the -(CH₂)ₙ₁- group is bound to the phenyl ring through the [C]₂ or the [C]₃ or the [C]₄; or
   n, n2, n5 are 1,
   n3 and n4 are equal to 0, and
   n1 is an integer from 1 to 10,
   Y¹ is -(CH₂)ₙₐ-CH=CH- wherein na is 0,
   X₁ is WC(O)- wherein W is oxygen and the WC(O) group is bound to the phenyl ring through the [C]₄,
   R⁴ is CH₃ and the (OR⁴) group is bound to the phenyl ring through the [C]₃;
d) 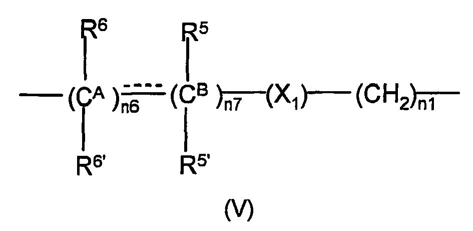 wherein
   the -ONO₂ is bound to the -(CH₂)ₙ₁- group;
   n1 is an integer from 1 to 10, n6 and n7 are 1, X₁ is WC(O)- wherein W is sulfur,
   R⁵, R^{5'} and R^{6'} are H,
   R⁶ is NHCOCH₃.

Another group of preferred compounds are those of formula (I) wherein s is 1,
A is a β-adrenergic blocker residue of formula (II) as above defined,
Z₁ is H and Z is -C(O)-, and
the bivalent radical Y have the following meaning:
c) 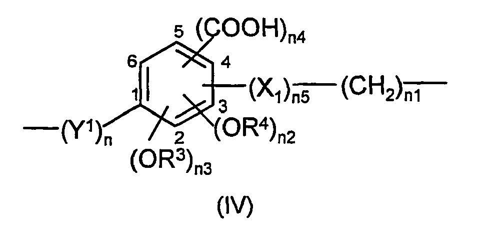 wherein the -ONO₂ group is bound to (CH₂)ₙ₁;
   n, n2, n3, n4, n5 are equal to 0,
   n1 is 1 and the -(CH₂)ₙ₁- group is bound to the phenyl ring through the [C]₂ or the [C]₃ or the [C]₄; or
   n, n2, n5 are 1,
   n3 and n4 are equal to 0, and
   n1 is an integer from 1 to 10,
   Y¹ is -(CH₂)ₙₐ-CH=CH- wherein na is 0,
   X₁ is -WC(O)- wherein W is oxygen and the WC(O) group is bound to the phenyl ring through the [C]₄,
   R⁴ is CH₃ and the (OR⁴) group is bound to the phenyl ring through the [C]₃;
d) 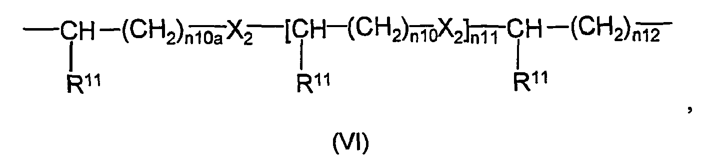 wherein
   X₂ is O or S, and n10a and n11 are 0, n12 is 1 and R¹¹ is H and the -ONO₂ group is bound to(CH₂)ₙ₁₂.

Another group of preferred compounds are those of formula (I) wherein s is 2,
A is a β-adrenergic blocker residue of formula (II) as above defined,
Z₁ and Z are -C(O)-, and
the bivalent radical Y have the following meaning:
a) straight C₁-C₁₀ alkylene, preferably C₃-C₆ alkylene;
c) 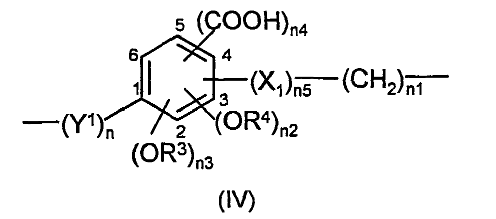 wherein the -ONO₂ group is bound to (CH₂)ₙ₁;
   n, n2, n3, n4, n5 are equal to 0,
   n1 is 1 and the -(CH₂)ₙ₁- group is bound to the phenyl ring through the [C]₂ or the [C]₃ or the [C]₄;
   or n, n2, n5 are 1,
   n3 and n4 are equal to 0, and
   n1 is an integer from 1 to 10,
   Y¹ is -(CH₂)ₙₐ-CH=CH- wherein na is 0,
   X₁ is -WC(O)- wherein W is oxygen and the WC(O) group is bound to the phenyl ring through the [C]₄,
   R⁴ is CH₃ and the (OR⁴) group is bound to the phenyl ring through the [C]₃;
d) 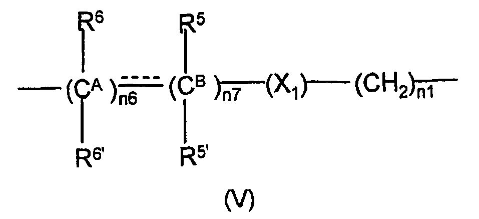 wherein
   the -ONO₂ is bound to the -(CH₂)ₙ₁- group;
   n1 is an integer from 1 to 10,
   n6 and n7 are 1,
   X₁ is -WC(O)- wherein W is sulfur,
   R⁵, R^{5'} and R^{6'} are H, R⁶ is NHCOCH₃.

Preferred compounds of formula (I) according to the present invention are the following:

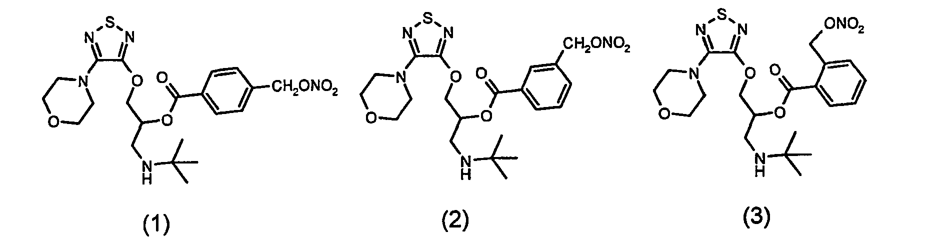

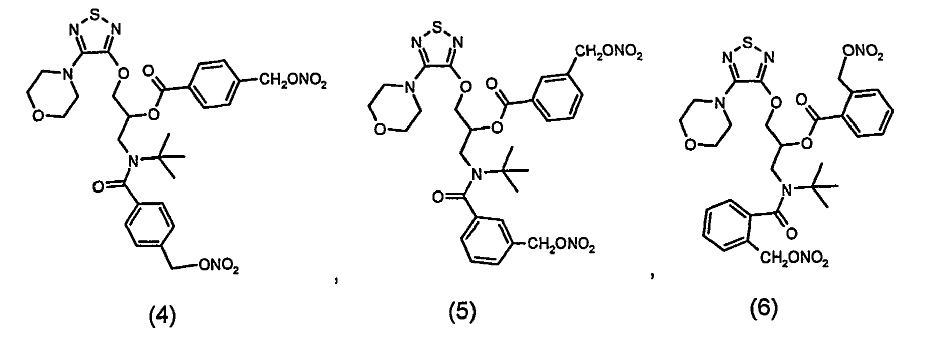

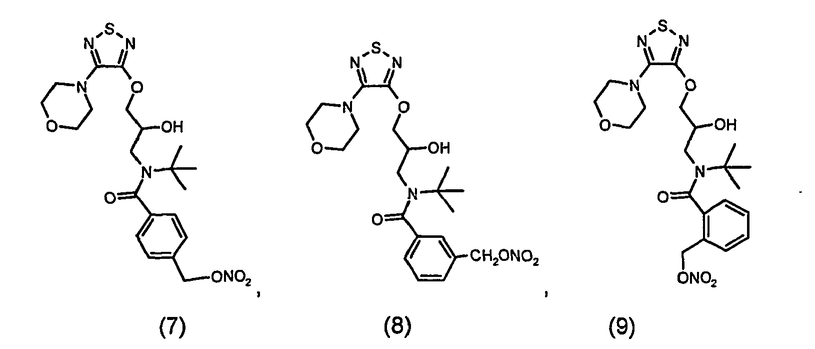

Examples of "straight or branched C₁-C₂₀ alkylene" include, but are not limited to, methylene, ethylene, propylene, isopropylene, n-butylene, pentylene, n-hexylene and the like.

As stated above, the invention includes also the pharmaceutically acceptable salts of the compounds of formula (I) and stereoisomers thereof.

Examples of pharmaceutically acceptable salts are either those with inorganic bases, such as sodium, potassium, calcium and aluminium hydroxides, or with organic bases, such as lysine, arginine, triethylamine, dibenzylamine, piperidine and other acceptable organic amines.

The compounds according to the present invention, when they contain in the molecule one salifiable nitrogen atom, can be transformed into the corresponding salts by reaction in an organic solvent such as acetonitrile, tetrahydrofuran with the corresponding organic or inorganic acids.

Examples of pharmaceutical acceptable organic acids are: oxalic, tartaric, maleic, succinic, citric acids. Examples of pharmaceutical acceptable inorganic acids are: nitric, hydrochloric, sulphuric, phosphoric acids. Salts with nitric acid are preferred.

The compounds of the invention which have one or more asymmetric carbon atoms can exist as optically pure enantiomers, pure diastereomers, enantiomers mixtures, diastereomers mixtures, enantiomer racemic mixtures, racemates or racemate mixtures. Within the object of the invention are also all the possible isomers, stereoisomers and their mixtures of the compounds of formula (I).

The compounds and compositions of the present invention can be administered by any available and effective delivery system including but not limited to, orally, bucally, parenterally, by inhalation spray, by topicall application, by injection, transdermally, or rectally (e.g. by the use of suppositories) in dosage unit formulations containing conventional nontoxic pharmaceutically acceptable carriers, adjuvants, and vehicles.

Parenteral includes subcutaneous injections, intravenous, intramuscular, intrasternal injection, or infusion technique.

Solid dosage forms for oral administration can include for example capsule, tablets, pills, powders, granules and gel. In such solid dosage forms, the active compounds can be admixed with at least one inert diluent such as sucrose, lactose or starch. Such dosage form can also comprise, as normal practice, additional substance other than inert diluent, e.g., lubricating agent such as magnesium stearate.

Injectable preparations, for example sterile injectable aqueous or oleaginous suspensions can be formulated according to the known art using suitable dispersing agents, wetting agents and/or suspending agents.

The composition of this invention can further include conventional excipients, i.e., pharmaceutical acceptable organic or inorganic substances which do not deleteriously react with the active compounds.

The doses of β-adrenergic blockers nitrooxyderivatives can be determinated by standard clinique technique and are in the same ranges or less than as described for commercially available compounds as reported in the: Physician's Desk Reference, Medical Economics Company, Inc., Oradell, N.J., 58th Ed., 2004; The pharmacological basis of therapeutics, Goodman and Gilman, J. G. Hardman, L. e. Limbird, 20th Ed.

### EXPERIMENTAL: synthesis procedure

The compounds of the invention can be synthesized as shown in Schemes 1 to 6. The compounds of general formula (I) A-(Y-ONO₂)ₛ, defined in Schemes 1-3 as compounds of formula D, wherein s is 1, Y is as above defined and A is a β-adrenergic blocker residue of formula (II), wherein Z is -C(O)- and Z₁ is H, the enantiomers, diastereoisomer and a pharmaceutically acceptable salt thereof, can be prepared as outlined in Schemes 1 -3.

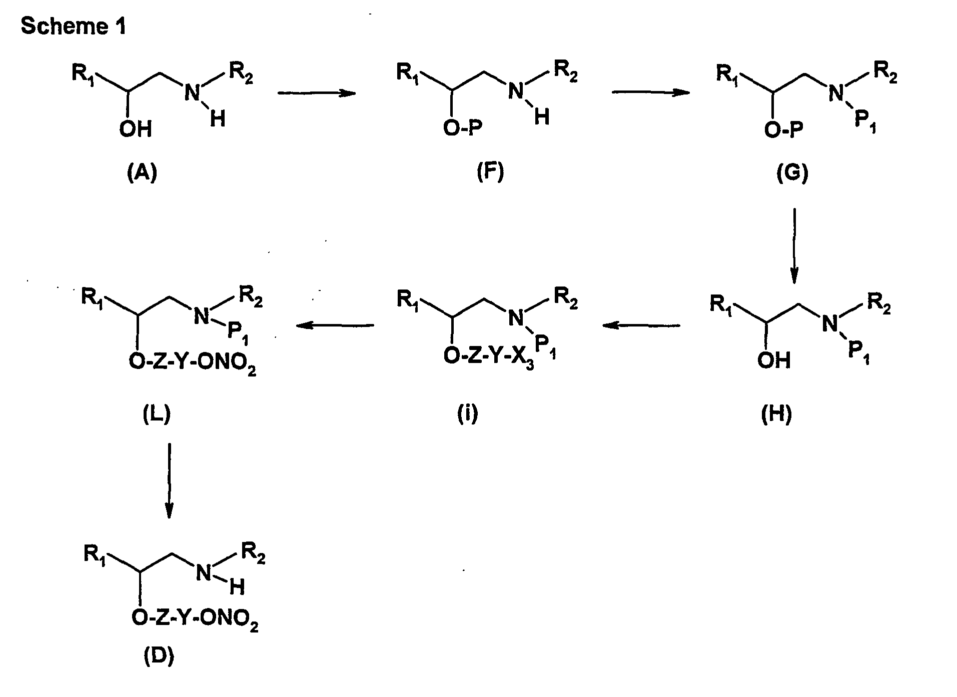

Compounds of formula (i) wherein R₁, R₂, Z and Y are as above defined, P₁ is an amine protecting group such as tert-butyloxycarbonyl ester (t-Boc) and X₃ is an halogen atom preferably Cl, Br and I, are converted to compounds of formula (L) wherein R₁, R₂, P₁, Z and Y are as above defined, by reaction with AgNO₃ in a suitable organic solvent such as acetonitrile, tetrahydrofurane, a silver nitrate molar excess is preferably used and the reaction is carried out, in the dark, at a temperature from room temperature to the boiling temperature of the solvent. The compounds of formula (L) are converted to the compounds of formula (D) by deprotecting the amine group (strong acid, such as HCl in dioxane or trifluoroacetic acid, is used to remove a t-butyl carbamate). Other preferred methods for removing the amine protecting groups are those described in T. W. Greene "Protective groups in organic synthesis", Harvard University Press, 1980.
The compounds of formula (H) wherein R₁, R₂, Z, P₁ and Y are as above defined, are converted to the esters of formula (i) wherein R₁, R₂, Y, Z, X₃ and P₁ are as above defined, by reaction with an appropriate acid (Q1) of formula X₃-Y-COOH wherein Y and X₃ are as above defined. The reaction is generally carried out in an inert organic solvent such as N,N'-dimethylformamide, tetrahydrofuran, benzene, toluene, dioxane, a polyhalogenated aliphatic hydrocarbon at a temperature from 0°C to 50°C in presence of a dehydrating agent such as dycyclohexylcarbodiimide DCC or 1-ethyl-3-(3-dimethylaminopropyl)carbodiimide hydrochloride (EDAC HCl) with a catalyst, such as 4-N,N-dimethylaminopyridine (DMAP).
The compounds of formula (H) wherein R₁, R₂ and P₁ are as above defined, can be obtained by deprotecting the hydroxylic group of the compounds of formula (G) wherein R₁, R₂ are as above defined and P is a hydroxylic protecting group such as silyl ethers, such as trimethylsilyl or tert-butyl-dimethylsilyl and those described in T. W. Greene "Protective groups in organic synthesis", Harvard University Press, 1980. Fluoride ion is the preferred method for removing silyl ether protecting group.
The compounds of formula (G) wherein R₁, R₂, P and P₁ are as above defined, can be obtained by reacting the compounds of formula (F) wherein R₁, R₂ and P are as above defined with a suitable amine protecting group (P₁) as above described.
The alcohol group of the compounds of formula (A) wherein R₁, R₂ are as above defined, is protected to afford the compounds of formula (F) wherein R₁, R₂ are as above defined Preferred protecting groups for the alcohol moiety are silyl ethers, such as trimethylsilyl or tert-butyl-dimethylsilyl.
The compounds (A) wherein R₁, R₂ are as above defined are commercially available, the acids of formula X₃-Y-COOH wherein X₃ is as above defined, are commercially available.

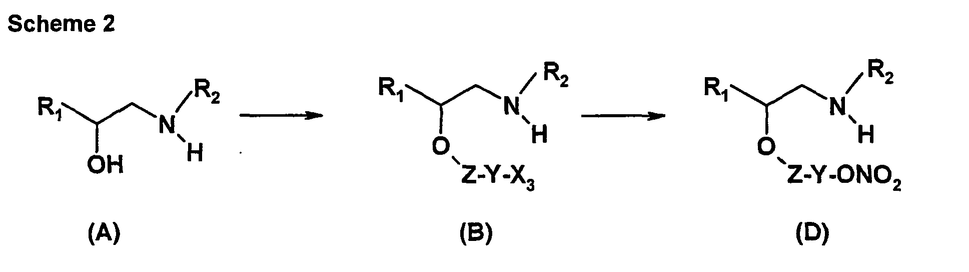

Compounds of formula (B) wherein R₁, R₂, Z, Y are as above defined and X₃ is an halogen atom, such as Cl**,** Br and I, are converted to compounds of formula (D) wherein R₁, R₂, Z and Y are as above defined, by reaction with AgNO₃ in a suitable organic solvent such as acetonitrile, tetrahydrofurane, a silver nitrate molar excess is preferably used and the reaction is carried out, in the dark, at a temperature from room temperature and the boiling temperature of the solvent.
The compounds of formula (B) wherein R₁, R₂, Z, Y and X₃ are as above defined can be obtained by reaction of compounds of formula (A) with an appropriate acid chloride (Q) of formula X₃-Y-C(O)Cl, wherein X₃ is chosen among chlorine, bromine, and Y is as above defined. The reaction of formation of the ester is carried out in an inert organic solvent such as N,N'-dimethylformamide, tetrahydrofuran, benzene, toluene, chloroform in presence of a base as triethylamine, pyridine at a temperature from room temperature and 50°C. The reaction is completed within a time range from 30 minutes to 24 hours.

Alternatively the compounds of formula (B) can be obtained by reaction of a compound of formula (A) with an acid (Q1) of formula X₃-Y-C(O)OH in the presence of a dehydrating agent as dicyclohexylcarbodiimide (DCC) or N'-(3-dimethylaminopropyl)-N-ethylcarbodiimide hydrochloride (EDAC) and a catalyst, such as N,N-dimethylamino pyridine. The reaction is carried out in an inert organic solvent such as N,N'-dimethylformamide, tetrahydrofuran, benzene, toluene, dioxane, a polyhalogenated aliphatic hydrocarbon at a temperature from 0°C and 50°C. The reaction is completed within a time range from 30 minutes to 36 hours.

The compounds of formula (Q1), where X₃ is an halogen atom are commercially available or can be obtained from the corresponding commercially available hydroxyl acid by well known reactions, for example by reaction with thionyl or oxalyl chloride, halides of P^{III} or P^{V} in solvents inert such as toluene, chloroform, DMF, etc.

The compounds (A) wherein R₁, R₂ are as above defined are commercially available.

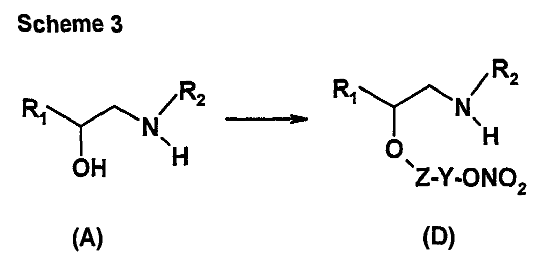

Alternatively the compounds of formula (D) can be obtained as described below. The compounds of formula A are converted to the ester (D) by reaction of the alcohol group with a nitrooxyderivative, containing activated acylating group, of formula Cl(O)C-Y-ONO_{2.} The nitrooxy compounds can be obtained from the corresponding alcohols of formula Cl(O)C-Y-OH by reaction with nitric acid and acetic anhydride in a temperature range from -50°C to 0°C or from the corresponding halogen derivatives of formula Cl(O)C-Y-Hal by reaction with silver nitrate in the presence of an inert solvent such as acetonitrile, tetrahydrofurane. A silver nitrate molar excess is preferably used and the reaction is carried out, in the dark, a temperature from the boiling temperature and room temperature. The reaction is completed within a time range from 30 minutes to 3 days.

The compounds of general formula (I) A-(Y-ONO₂)ₛ, defined in Scheme 4 as compounds of formula (D1), wherein s is 1, Y is as above defined and A is a β-adrenergic blocker residue of formula (II), wherein Z is -C(O)O- and Z₁ is H, the enantiomers, diastereoisomer and a pharmaceutically acceptable salt thereof, can be prepared as outlined in Scheme 4.

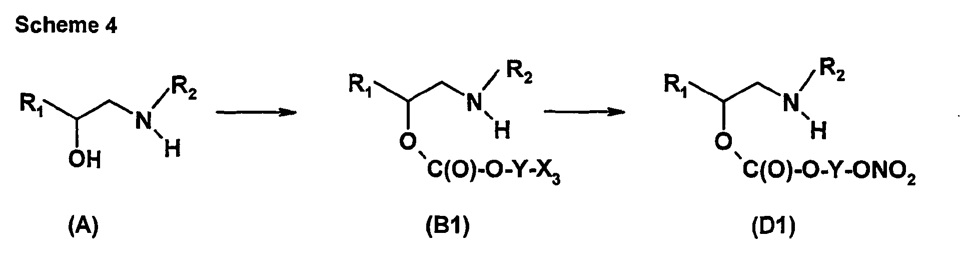

The compounds of formula (B1) wherein R₁, R₂, Y are as above defined and X₃ is an halogen atom, such as Cl, Br and I, are converted to compounds of formula (D1) wherein R₁, R₂, and Y are as above defined, by reaction with AgNO₃ in a suitable organic solvent such as acetonitrile, tetrahydrofurane, a silver nitrate molar excess is preferably used and the reaction is carried out, in the dark, at a temperature from room temperature and the boiling temperature of the solvent.
The compounds of formula (A) wherein R₁ and R₂ are as above defined are converted to the compounds (B1) by reaction with an appropriate compound (Q2) having formula X₃-Y-OC(O)Cl wherein X₃ is Cl, Br or I, and Y is as defined above. The reaction is generally carried out in presence of a base in an aprotic polar or non-polar solvent such as THF or CH₂Cl₂ at temperatures range between 0°-65°C or in a double phase system H₂O/Et₂O at temperatures range between 20°- 40°C.
The compounds of formula (Q2) are commercially available or can be obtained from the corresponding alcohols by reaction with triphosgene in presence of an organic base.

The compounds of general formula (I) A-(Y-ONO₂)ₛ, defined in Scheme 5 as compounds of formula (D), wherein s is 1, Y is as above defined and A is a β-adrenergic blocker residue of formula (II), wherein Z is 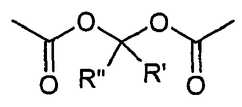 wherein R' and R" are as above defined and Z₁ is H, the enantiomers, diastereoisomer and a pharmaceutically acceptable salts thereof, may be prepared as outlined in Scheme 5:

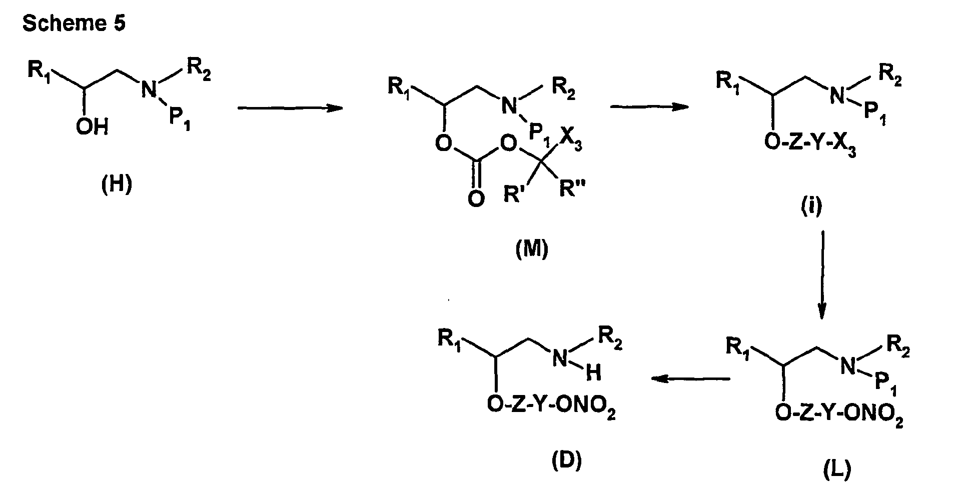

The compounds of formula (i) wherein R₁, R₂, Z and Y are as above defined, P₁ is an amine protecting group such as tert-butyloxycarbonyl ester (t-Boc) and X₃ is an halogen atom such as Cl, Br and I, are converted to compounds of formula (L) wherein R₁, R₂, P₁, Z and Y are as above defined, by reaction with AgNO₃ in a suitable organic solvent such as acetonitrile, tetrahydrofurane, a silver nitrate molar excess is preferably used and the reaction is carried out, in the dark, at a temperature from room temperature and the boiling temperature of the solvent. The compounds of formula (L) are converted to the compounds of formula (D) by deprotecting the amine group (strong acid, such as HCl in dioxane or trifluoroacetic acid, is used to remove a t-butyl carbamate). Other preferred methods for removing the amine protecting groups are those described in T. W. Greene "Protective groups in organic synthesis", Harvard University Press, 1980.
The compounds of formula (i) wherein R₁, R₂, Y, X₃, Z and P₁ are as above defined, can be obtained by reacting the compounds of formula (M) wherein R₁, R₂, P₁, R', R" and X₃ are as above defined, with an acid (Q1) of formula X₃-Y-COOH wherein X₃ is an halogen atom and Y is as above defined. The reaction is carried out in an inert organic solvent such as N,N'-dimethylformamide, tetrahydrofuran, benzene, toluene, dioxane, a polyhalogenated aliphatic hydrocarbon at a temperature from 0°C and 50°C in the presence of a dehydrating agent such as dycyclohexylcarbodiimide DCC or 1-ethyl-3-(3-dimethylaminopropyl)carbodiimide hydrochloride (EDAC HCl) with a catalyst, such as 4-N,N-dimethylaminopyridine (DMAP).
The reaction is complete within a time range from 30 minutes to 24 hours.
The compounds of formula (M) wherein R₁, R₂, P₁, R', R" and X₃ are as above defined, can be obtained by reacting compounds the of formula (H) with an acyl compound (S) of formula X₃-C(R')(R")-OC(O)X₃ wherein X₃ is an halogen atom. The reaction is carried out in presence of an organic or inorganic base in a polar solvent as DMF, THF, acetonitrile at a temperature in the range from -5°C to 60°C or in a double phase system according to methods well known in the literature.
The amine group of the compounds (A) is protected to afford the compounds of formula (H) wherein P₁ is a suitable amine protecting group such as tert-butyloxycarbonyl ester (t-Boc) The compounds (S) are commercially available.

The compounds of general formula (I) A-(Y-ONO₂)ₛ, defined in Scheme 6 as compounds of formula (E), wherein s is 2, Y is as above defined and A is a β-adrenergic blocker residue of formula (II), wherein Z₁ and Z are -C(O)-, the enantiomers, diastereoisomer and a pharmaceutically acceptable salt thereof, can be synthesized as shown in Scheme 6.

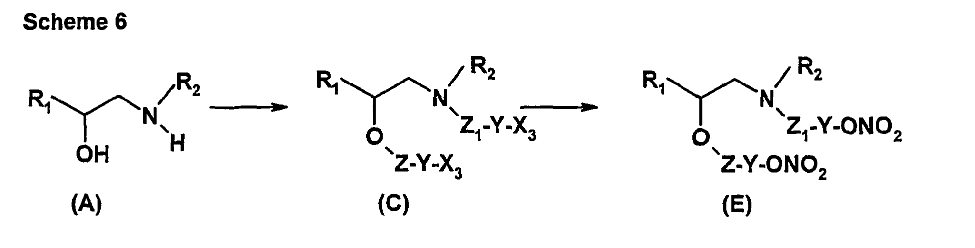

Compound of formula (C) wherein R₁, R₂, Z, Z₁ and Y are as above defined and X₃ is an halogen atom, such as Cl, Br and I, are converted to compounds of formula (E) wherein R₁, R₂, Z and Y are as above defined, by reaction with AgNO₃ in a suitable organic solvent such as acetonitrile, tetrahydrofurane, a silver nitrate molar excess is preferably used and the reaction is carried out, in the dark, at a temperature from room temperature and the boiling temperature of the solvent.
The compounds of formula (C) wherein R₁, R₂, Z, Z₁, Y and X₃ are as above defined can be obtained by reaction of compounds of formula (A) with an appropriate acid chloride (Q) of formula X₃-Y-C(O)Cl, wherein X₃ is chosen among chlorine, bromine, and Y is as above defined. The reaction is carried out in an inert organic solvent such as N,N'-dimethylformamide, tetrahydrofuran, benzene, toluene, chloroform in presence of a base as triethylamine, pyridine at a temperature from room temperature and 50°C. The reaction is completed within a time range from 30 minutes to 24 hours.
Alternatively the compounds of formula (C) can be obtained by reaction of compounds of formula (A) with an acid (Q1) of formula X₃-Y-COOH in the presence of a dehydrating agent such as dicyclohexylcarbodiimide (DCC) or N'-(3-dimethylaminopropyl)-N-ethylcarbodiimide hydrochloride (EDAC) and a catalytic amount of N,N-dimethylamino pyridine. The reaction is carried out in an inert organic solvent such as as N,N'-dimethylformamide, tetrahydrofuran, benzene, toluene, dioxane, a polyhalogenated aliphatic hydrocarbon at a temperature from 0°C and 50°C. The reaction is completed within a time range from 30 minutes to 36 hours .
The compounds of formula (Q1), where X₃ is an halogen atom are commercially available or can be obtained from the corresponding commercially available hydroxyl acids by well known reactions, for example by reaction with thionyl or oxalyl chloride, halides of P^{III} or P^{V} in solvents inert such as toluene, chloroform, DMF, etc.
The compounds (A) wherein R₁, R₂ are as above defined are commercially available.
The compounds of formula (D) can also be obtained as described below. The compounds of formula A are converted to the compounds (E) by reaction with a nitrooxy derivative of formula Cl(O)C-Y-ONO₂ containing an activated acylating group.
The nitrooxy-compounds can be obtained from the corresponding alcohols of formula Cl(O)C-Y-OH by reaction with nitric acid and acetic anhydride in a temperature range from -50°C to 0°C or from the corresponding halogen derivatives of formula Cl(O)C-Y-Hal by reaction with silver nitrate in the presence of an inert solvent such as acetonitrile, tetrahydrofurane. A silver nitrate molar excess is preferably used and the reaction is carried out, in the dark, a temperature from the boiling temperature and room temperature. The reaction is completed within a time range from 30 minutes to 3 days.

### EXAMPLES

The following non-limiting examples further describe and enable of ordinary skilled in the art to make and use the present invention.

### Example 1

### 4-(Nitrooxymethyl)benzoic acid (S)-1-[(1,1-dimethylethyl)amino]-3-[[4-(4-morpholinyl)-1,2,5-thiadiazol-3-yl]oxy]-2-propanoate maleate salt

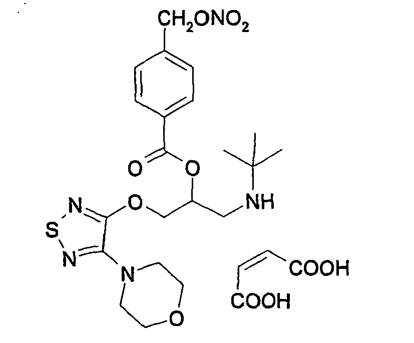

### 1a. 4-(Chloromethyl)benzoic acid (S)-1-[(1,1-dimethylethyl)amino]-3-[[4-(4-morpholinyl)-1,2,5-thiadiazol-3-yl]oxy]-2-propanoate

To a solution of timolol (3.5g, 11mmol) in chloroform (200ml) 4-chloromethyl benzoic acid (1.9g, 11mmol), EDAC (3.16g, 16.5mmol) and N,N-dimethylaminopyridine (catalytic amount) were added. The reaction was stirred for 12 hours at room temperature. The solution was washed with water, dried over sodium sulphate and concentrated under reduced pressure. The residue was purified by flash chromatography eluting with chloroform/isopropanol 10/0.5 to give the title compound 3g as a white powder.

### 1b. 4-(Nitrooxymethyl)benzoic acid (S)-1-[(1,1-dimethylethyl)amino]-3-[[4-(4-morpholinyl)-1,2,5-thiadiazol-3-yl]oxy]-2-propanoate

A solution of the product of example 1a (1g, 2.1mmol) and silver nitrate (0.71g, 4.21 mmol) in acetonitrile (50ml) was stirred at 60°C, in the dark, for 36 hours. The precipitated (silver salts) was removed by filtration and the solvent was evaporated under vacuum. The residue was treated with chloroform and water. The organic layer was dried over sodium sulfate and the solvent was evaporeted. The residue was purified by flash chromatography, eluting with chloroform/isopropanol 10/0.5 to give the title compound 0.6g as white powder.

### 1c. 4-(Nitrooxymethyl)benzoic acid (S)-1-[(1,1-dimethylethyl)amino]-3-[[4-(4-morpholinyl)-1,2,5-thiadiazol-3-yl]oxy]-2-propanoate maleate salt

To a solution of the product of the example 1b (0.6g, 1.2mmol) in acetone (100ml) maleic acid (0.14g, 1.2mmol) was added. The reaction was stirred at room temperature for 1 hours. The precipitated was filtered, washed with acetone and dried under vacuum to afford the title compound 0.6g as a white powder.
M.p.= 160°C
¹H-NMR (CDCl₃) δ (ppm): 7.99 (2H,d); 7.42 (2H,d); 5.93 (2H,s); 5.87 (1H,m); 5.46 (2H,s); 4.82 (1H,dd); 4.71 (1H,dd); 3.73 (4H,m); 3.44 (4H,m);1.49 (9H,s).

### Example 2

### 4-(Nitrooxymethyl)benzoic acid (S)-1-[(1,1-dimethylethyl)[(4-nitrooxymethyl)benzoyl] amino]-3-[[4-(4-morpholinyl)-1,2,5-thiadiazol-3-yl]oxy]-2-propanoate

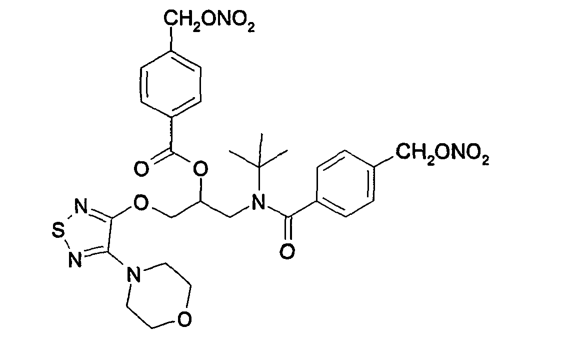

### 2a. 4-(Chloromethyl)benzoic acid (S)-1-[(1,1-dimethylethyl)[(4-chloromethyl)benzoyl] amino]-3-[[4-(4-morpholinyl)-1,2,5-thiadiazol-3-yl]oxy]-2-propanoate

To a solution of timolol hydrocloride (8g, 22,66mmol) in chloroform (130ml) a mixture of 4-chloromethyl benzoylchloride (4,28g, 22,66mmol) and triethylamine (6.2ml, 44.66mmol) in chloroform (70ml) was added dropwise. The reaction was stirred for 24 hours at room temperature. The solution was treated with water and diethyl ether, the organic layers were dried over sodium sulfate and concentrated under reduced pressure. The residue was purified by flash chromatography, eluting with chloroform/isopropanol 10/0.3 to give the title compound 3g as powder.

### 2b. 4-(Nitrooxymethyl)benzoic acid (S)-1-[(1,1-dimethylethyl)[(4-nitrooxymethyl)benzoyl] amino]-3-[[4-(4-morpholinyl)-1,2,5-thiadiazol-3-yl]oxy]-2-propanoate

A solution of the product of example 2a (1.5g, 2.4mmol) and silver nitrate (1.23g, 7.2mmol) in acetonitrile (100ml) was stirred at 60°C, in the dark, for 36 hours. The precipitated (silver salts) was removed by filtration and the filtrate was concentrated. The residue was treated with chloroform and water and the organic layer was dried over sodium sulfate and concentrated under reduced pressure. The residue was purified by flash chromatography eluting with chloroform/isopropanol 10/0.2 to give the title product 0.95g as a yellow powder.
M.p.= 44-46°C
¹H-NMR (CDCl₃) δ (ppm): 7.95 (2H,d); 7.50 (2H,d); 7.38(4H,s); 5.79 (1H,m); 5.75(2H,s), 5.74 (2H,s); 4.50 (1H,dd); 4.30 (1H,dd); 3.95 (1H,dd); 3.85 (1H,dd); 3.59 (4H,m); 3.34 (4H,m); 1.60 (9H,s).

### Example 3

### (S)-1-[(1,1-dimethylethyl)[(4-nitrooxymethyl)benzoyl]amino]-3-[[4-(4-morpholinyl)-1,2,5-thiadiazol-3-yl]oxy]-2-propanol

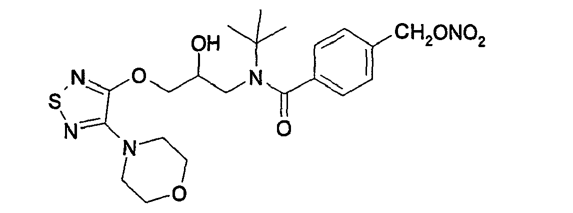

### 3a. (S)-1-[(1,1-dimethylethyl)amino]-3-[[4-(4-morpholinyl)-1,2,5-thiadiazol-3-yl]oxy]-2-propan-1-tert-butyldimethylsilylether

To a solution of timolol (2g, 6,32mmol) in N,N-dimethylformamide (10ml) tert-butyldimethylsilylchloride (1,15g, 7,58mmol) and imidazole (1g, 15,8mmol) were added. The reaction was stirred for 2 hours at room temperature. The solution was concentrated under reduced pressure and the residue was purified by flash chromatography eluting with chloroform/isopropanol 10/0.3 to give the title compound 1,5g.

### 3b. (S)-1-[(1,1-dimethylethyl)[(4-chloromethyl)benzoyl]amino]-3-[[4-(4-morpholinyl)-1,2,5-thiadiazol-3-yl]oxy]-2-propan-1- tert-butyldimethylsilylether

To a solution of the product of the example 3a (0,7g, 1,62mmol) in chloroform (50ml) 4-chloromethyl benzoylchloride (0,46g, 2,44mmol) and triethylamine (0,39ml, 2,44mmol) were added. The reaction was stirred for 24 hours at room temperature. The solution was treated with water and diethyl ether, the organic layers were dried over sodium sulphate and concentrated under reduced pressure. The residue was purified by flash chromatography eluting with n-hexanelethyl acetate 7/3 to give the title product (0,7g).

### 3c. (S)-1-[(1,1-dimethylethyl)[(4-chloromethyl)benzoyl] amino]-3-[[4-(4-morpholinyl)-1,2,5-thiadiazol-3-yl]oxy]-2-propanol

To a solution of the product of example 3b (0,6g, 1,03mmol) in tetrahydrofuran (50ml) cooled at 0°C, a solution of tetrabutylamonium floride in tetrahydrofuran 1 M (0,54ml, 2,05mmol) was added. The reaction was stirred for 30 minutes at room temperature. The solution was concentrated under reduced pressure and the residue was purified by flash chromatography eluting with n-hexane/ethyl acetate 1/1 to give the title product 0,2g.

### 3d. (S)-1-[(1,1-dimethylethyl)[(4-nitrooxymethyl)benzoyl] amino]-3-[[4-(4-morpholinyl)-1,2,5-thiadiazol-3-yl]oxy]-2-propanol

A solution of the product of example 3c (0,15g, 0,32mmol) and silver nitrate (0,11g, 0,64mmol) in acetonitrile (50ml) was stirred at 65°C, in the dark, for 32 hours. The precipitated (silver salts) was removed by filtration and the filtrate concentrate. The residue was treated with methylene chloride and water and the organic layer was dried over sodium sulfate and concentrated under reduced pressure. The residue was purified by flash chromatography eluting with n-hexane/ethyl acetate 45/65 to afford the title compound 0.65g as a white powder.
M.p.= 50-54°C
¹H-NMR (CDCl₃) δ (ppm): 7.40 (4H,s); 5.44 (2H,s); 4.33-4.18(3H,m), 3.79 (4H,dd); 3.64-3.50 ( (2H,m); 4.46 (4H,dd); 3.00 (1 H,s); 1.53 (9H,s).

### Example 4

### Measurements of cGMP in rat PC12 cell line.

cGMP contributes to the function and interaction of several vascular cell types and its dysfunction is involved in major cardiovascular diseases such as hypertension, diabetic complications, atherosclerosis, and tissue infarction. Therefore the extent of cGMP formation elicited by the compounds of the inventions was evaluated in the rat pheochromocytoma (PC12) cell line.

### Tested compounds

**1)** Timolol (parent compound)
**2)** 4-(Nitrooxymethyl)benzoic acid (S)-1-[(1,1-dimethylethyl)[(4-nitrooxymethyl)benzoyl] amino]-3-[[4-(4-morpholinyl)-1,2,5-thiadiazol-3-yl]oxy]-2-propanoate (compound of example 1)
**3)** 4-(Nitrooxymethyl)benzoic acid (S)-1-[(1,1-dimethylethyl)[(4-nitrooxymethyl)benzoyl] amino]-3-[[4-(4-morpholinyl)-1,2,5-thiadiazol-3-yl]oxy]-2-propanoate (compound of example 2)
**4)** (S)-1-[(1,1-dimethylethyl)[(4-nitrooxymethyl)benzoyl]amino]-3-[[4-(4-morpholinyl)-1,2,5-thiadiazol-3-yl]oxy]-2-propanol (compound of example 3)

### Method

Cells were maintained at 37°C in DMEM medium enriched with 10% horse serum and 5% foetal bovine serum under 5% CO₂ atmosphere. At the time of experiments the cells were washed once with Hank's Balanced Salt Solution (HBSS) supplemented with 0.05% ascorbic acid and preincubated in the same buffer for 10 min in a floating water bath. After the preincubation step, cells were exposed for additional 45 min to either control conditions or increasing concentrations of test compounds ranging from 0.1 to 25 µM, in the presence of the phosphodiesterase inhibitor, IBMX (100 µM) and the NO-independent activator of soluble guanylyl cyclase, YC-1 (20 µM). The reaction was terminated by the removal of the incubating buffer and consecutive addition of 100 µl of absolute ethanol. The organic extracts were then evaporated to dryness and the residues dissolved in aqueous buffer for quantitative determination of intracellular cGMP levels using the cGMP enzyme immunoassay kit.
The obtained results reported in Table 1 are expressed as EC₅₀ (µM) and efficacy Emax (% of vehicle). As shown in the table the nitroderivatives of timolol elicited consistent increase of intracellular cGMP formation in PC12 cell line. Conversely, this effect was not shared by the parent compound.

**Table 1. Effects of nitroxyderivatives of timolol and ann of timolol on cGAMP formation in PC12 cells**

| **Compound** | **EC₅₀ (µM)** | **Eₘₐₓ (% of vehicle)** |
|---|---|---|
| Timolol | Not effective | Not effective |
| Compound of example 2 | 1.3 | 480 |
| Compound of example 1 | 12.6 | 796 |
| Compound of example 3 | 18.5 | 866 |

## Claims

1. A compound of general formula (I) A-(Y-ONO₂)ₛ and the enantiomers, diastereoisomers and pharmaceutically acceptable salts thereof, wherein
s is an integer equal to 1 or 2;
A is selected from the following β-adrenergic blockers residues of formula (II): 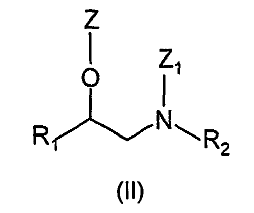 wherein
R₁ is selected from the group consisting off: 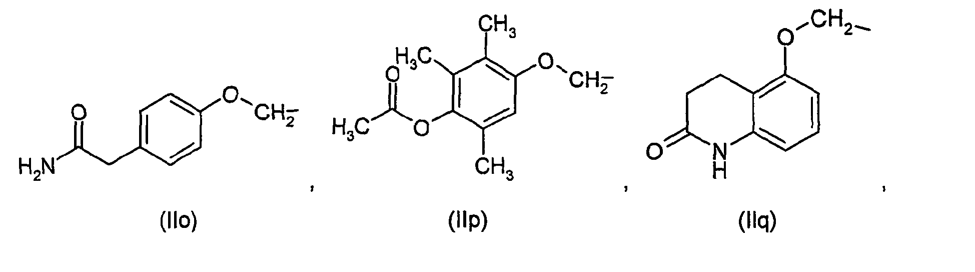 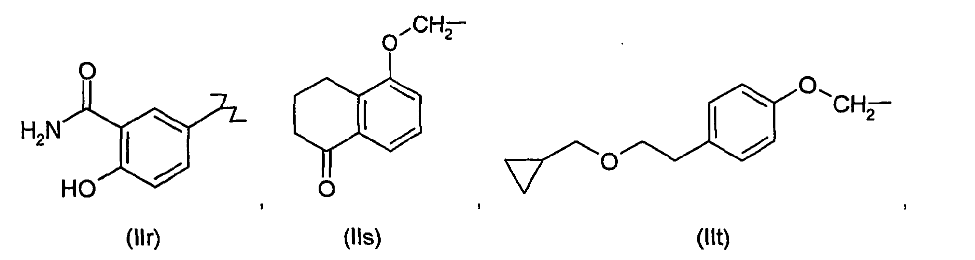 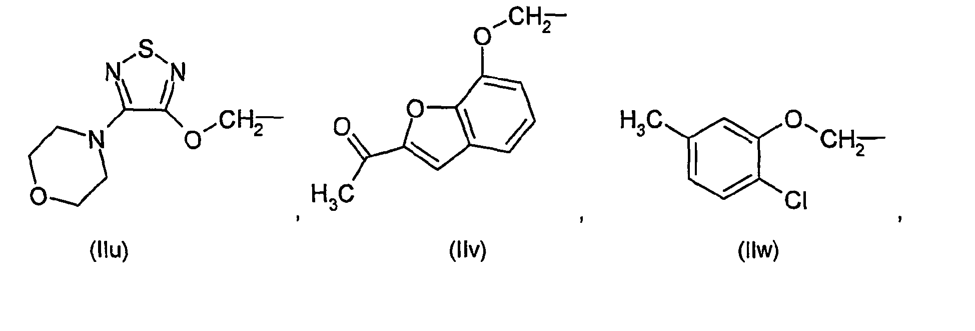 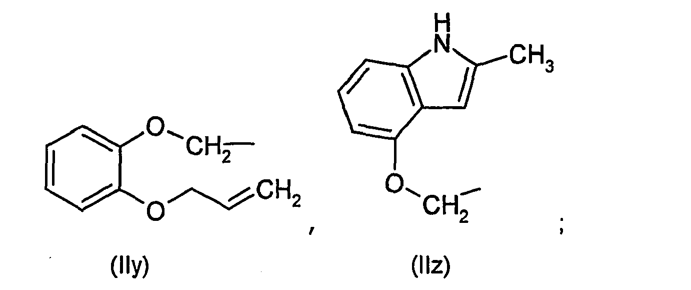 R₂ is selected from the group consisting of: -CH(CH₃)₂, -C(CH₃)₃ or 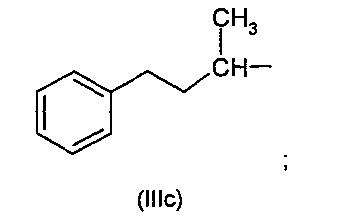 when the radical R₁ has chosen from the formulae (IIo), (IIp), (IIt), (IIu), (IIv), (IIy) or (IIz),
R₂ is -CH(CH₃)₂;
when the radical R₁ has chosen from the formulae (IIq), (IIs) or (IIw), R₂ is -C(CH₃)₃;
when the radical R₁ is (IIr), R₂ is (IIc);
Z is H or is a group capable of binding Y selected from the group consisting of: -C(O)-,
-C(O)O- or 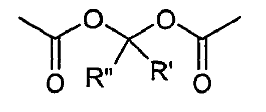 wherein R' and R" are the same or different, and are H or straight or branched C₁-C₄ alkyl;
Z₁ is H or a -C(O)-group capable of binding Y;
with the proviso that when s of formula (I) is 1 Z or Z₁ is H;
Y is a bivalent radical having the following meaning:
a)
- straight or branched C₁-C₂₀ alkylene being optionally substituted with one or more of the substituents selected from the group consisting of halogen atoms, hydroxy, -ONO₂ or T, wherein T is -OC(O)(C₁-C₁₀alkyl)-ONO₂, -O(C₁-C₁₀alkyl)-ONO₂;
b)
- cycloalkylene with 5 to 7 carbon atoms into cycloalkylene ring, the ring being optionally substituted with side chains T₁, wherein T₁ is straight or branched alkyl with from 1 to 10 carbon atoms;
c) 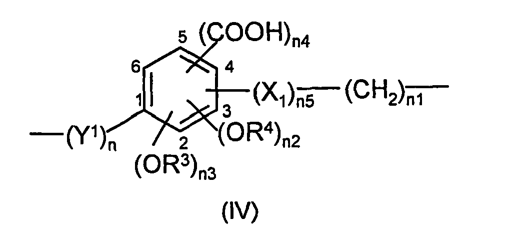 wherein:
n is an integer from 0 to 20, and n1 is an integer from 1 to 20;
n2, n3, n4 and n5 are integers equal or different from each other, equal to 0 or 1;
R³ and R⁴ are independently selected from H or CH₃;
Y¹ is -CH₂- or -(CH₂)ₙₐ-CH=CH- wherein na is an integer from 0 to 20;
X₁ is -WC(O)- or-C(O)W-, wherein W is oxygen, sulfur or NH;
with the proviso that:
- when s of formula (I) is 1, Z is -(CO)- and in formula (IV) of the bivalent radical Y n2, n3, n4, n5 are equal to 0 then n is 0 and n1 is 1;
- when s of formula (I) is 1, Z is -(CO)- and in formula (IV) of the bivalent radical Y n2, n3, n5 are equal to 0, n4 is 1 then n and n1 are different to 1;
d) 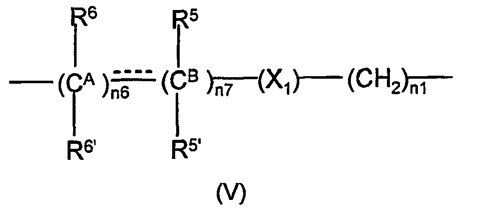 wherein:
n1 is an integer from 1 to 20;
X₁ is WC(O)- or-C(O)W-, wherein W is oxygen, sulfur or NH;
n6 is an integer from 1 to 20,
n7 is an integer from 0 to 20,
R⁵ and R^{5'} R⁶ and R^{6'} are independently selected from the group consisting of H, CH₃, OH, NH₂, NHCOCH₃, COOH, CH₂SH and C(CH₃)₂SH; when the bond between the C^{A} and C^{B} carbons is a double bond R⁵ and R⁶ or R^{6'} and R^{5'} are absent;
with the proviso that when Y is selected from the bivalent radicals mentioned under c)-d), the -ONO₂ group is linked to a -(CH₂)ₙ₁- group;
with the proviso that when s of formula (I) is 1 and Z is -(CO)- then the bivalent radical Y has not the meanings under a), b) and d);
e) 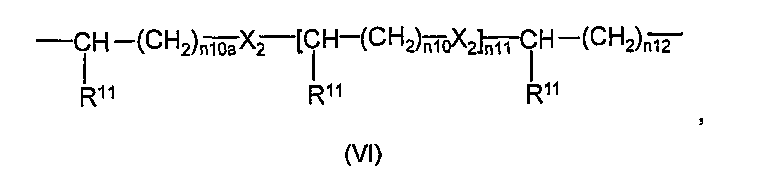 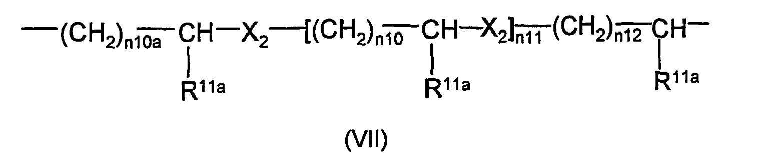 wherein X₂ is O or S,
n10a, n10 and n12 are integer independently selected from 0 to 20,
n11 is an integer from 0 to 6,
R¹¹ is H, CH₃ or nitrooxy group;
with the proviso that when in formula (I) s is 1, in formula (II) Z is -(CO)-, in formula (VI) of the bivalent radical Y n10a, n10, n12 are equal to 1 then X can not be an oxygen atom;
f) 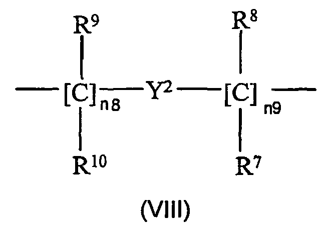
wherein:
n8 is an integer from 0 to 10;
n9 is an integer from 1 to 10;
R⁹, R¹⁰, R⁸, R⁷are the same or different, and are H or straight or branched C₁-C₄ alkyl;
wherein the -ONO₂ group is linked to 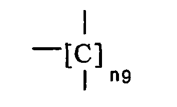
wherein n9 is as defined above;
Y² is an heterocyclic saturated, unsaturated or aromatic 5 or 6 members ring, containing one or more heteroatoms selected from nitrogen, oxygen, sulfur, and is selected from the group consisting of:

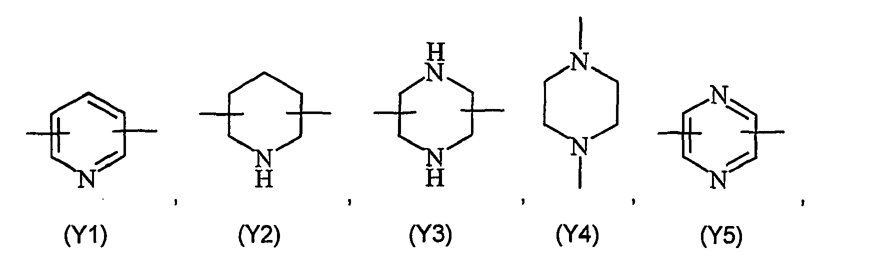

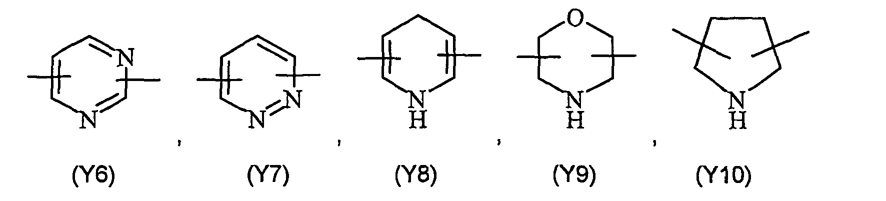

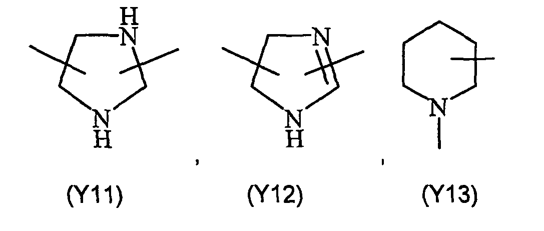

2. A compound and the enantiomers, diastereoisomers and pharmaceutically acceptable salts thereof according to claim 1 wherein s is equal to 1 and Z₁ is H.

3. A compound and the enantiomers, diastereoisomers and pharmaceutically acceptable salts thereof according to claim 2 wherein Z is -C(O)-.

4. A compound and enantiomers and diastereoisomers and pharmaceutically acceptable salts thereof according to claim 3 wherein
Y is 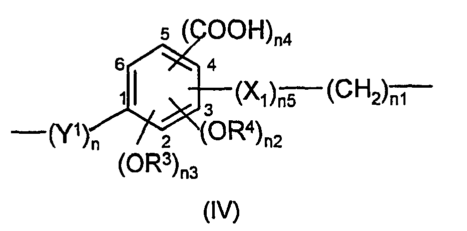 wherein
n, n2, n3, n4 and n5 are equal to 0
n1 is an integer equal to 1;

5. A compound and the enantiomers, diastereoisomers and pharmaceutically acceptable salts thereof according to claim 3 wherein
Y is 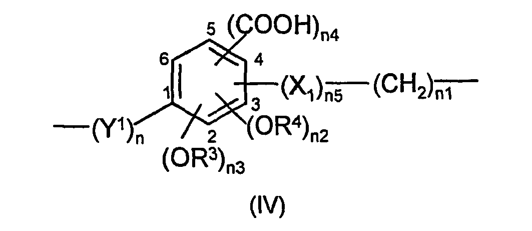 wherein
n, n2, n5 are 1,
n3 and n4 are equal to 0,
n1 is an integer from 1 to 10,
Y¹ is -(CH₂)ₙₐ-CH=CH- wherein na is 0,
X₁ is -WC(O)- wherein W is oxygen and X₁ is bound to the phenyl ring through the [C]₄,
R⁴ is CH₃ and the (OR⁴) group is bound to the phenyl ring through the [C]₃.

6. A compound and the enantiomers, diastereoisomers and pharmaceutically acceptable salts thereof according to claim 3 wherein
Y is 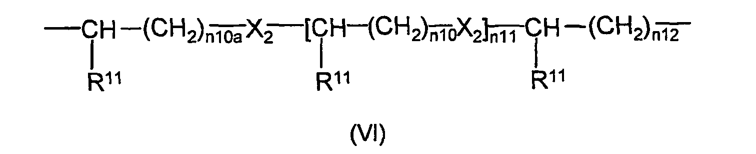 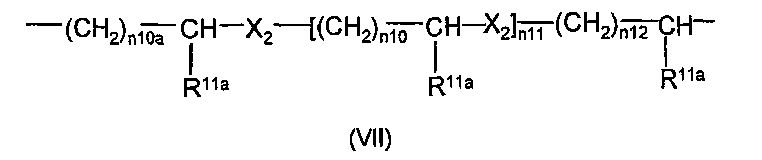 wherein
X₂ is O or S,
n10a, n10 and n12 are integers independently selected from 2 to 20;
n11 is an integer from 0 to 6;
R¹¹ is H, CH₃ or a nitrooxy group;
R^{11a} is CH₃ or a nitrooxy group.

7. A compound and the enantiomers, diastereoisomers and pharmaceutically acceptable salts thereof according to claim 2 wherein Z is -C(O)O-.

8. A compound and the enantiomers, diastereoisomers and pharmaceutically acceptable salts thereof according to claim 7 wherein
Y is a straight or branched C₁-C₂₀ alkylene being optionally substituted with one or more of the substituents selected from the group consisting of halogen atoms, hydroxy, -ONO₂ or T, wherein T is -OC(O)(C₁-C₁₀alkyl)-ONO₂, -O(C₁-C₁₀alkyl)-ONO₂.

9. A compound and the enantiomers, diastereoisomers and pharmaceutically acceptable salts thereof according to claim 8 wherein
Y is a straight or branched C₁-C₁₀ alkylene.

10. A compound and the enantiomers, diastereoisomers and pharmaceutically acceptable salts thereof according to claim 7 wherein
Y is 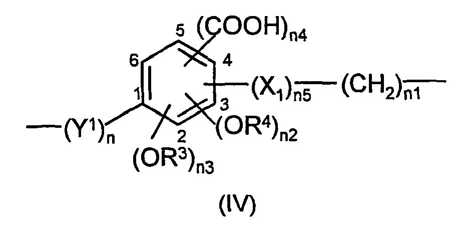 wherein
n is an integer from 0 to 20,
n1 is an integer from 1 to 20;
n2, n3, n4 and n5 are integers equal or different from each other, equal to 0 or 1;
R³ and R⁴ are independently selected from H or CH₃;
Y¹ is -CH₂- or -(CH₂)ₙₐ-CH=CH- wherein na is an integer from 0 to 20;
X₁ is WC(O)- or-C(O)W-, wherein W is oxygen, sulfur or NH.

11. A compound and the enantiomers, diastereoisomers and pharmaceutically acceptable salts thereof according to claim 10 wherein
n2, n3, n4, n5 are equal to 0,
n1 is 1,
n is an integer from 0 to 10,
Y¹ is CH₂.

12. A compound and the enantiomers, diastereoisomers and pharmaceutically acceptable salts thereof according to claim 7 wherein
Y is 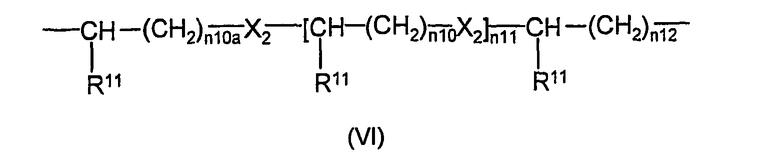 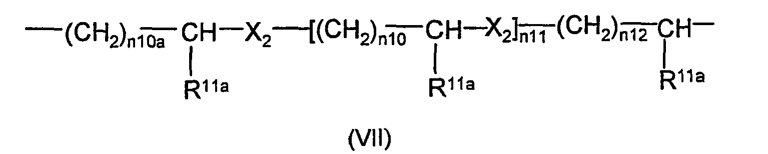 wherein
X₂ is O or S,
n10a, n10 and n12 are integers independently selected from 0 to 20;
n11 is an integer from 0 to 6;
R¹¹ is H, CH₃ or a nitrooxy group;
R^{11a} is CH₃ or a nitrooxy group.

13. A compound and the enantiomers, diastereoisomers and pharmaceutically acceptable salts thereof according to claim 12 wherein
Y is 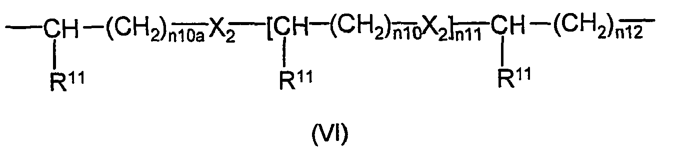 wherein
X₂ is O or S,
n10a and n11 are 0,
n12 is 1, and
R¹¹ is H;
wherein the -ONO₂ group is bound to the -(-CH₂)ₙ₁₂- group.

14. A compound and the enantiomers, diastereoisomers and pharmaceutically acceptable salts thereof according to claim 7 wherein
Y is 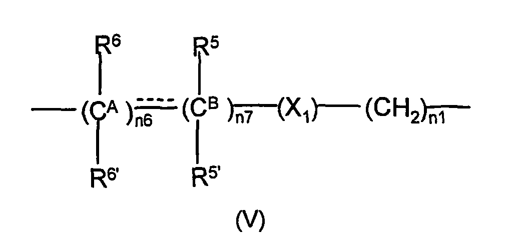 wherein:
n1 is an integer from 1 to 20;
X₁ is -WC(O)- or a -C(O)W-, wherein W is oxygen, sulfur or NH.
n6 is an integer from 1 to 20,
n7 is an integer from 0 to 20,
R⁵ and R^{5'} R⁶ and R^{6'} are independently selected from the group consisting of: H, CH₃, OH, NH₂, NHCOCH₃, COOH, CH₂SH and C(CH₃)₂SH; when the bond between the C^{A} and C^{B} carbons is a double bond R⁵ and R⁶ or R⁶' and R^{5'} are absent.

15. A compound and the enantiomers, diastereoisomers and pharmaceutically acceptable salts thereof according to claims 2 wherein Z is

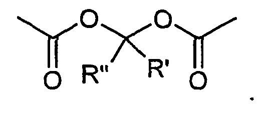

16. A compound and the enantiomers, diastereoisomers and pharmaceutically acceptable salts thereof according to claim 15 wherein
Y is a straight or branched C₁-C₂₀ alkylene being optionally substituted with one or more of the substituents selected from the group consisting of halogen atoms, hydroxy, -ONO₂ or T, wherein T is -OC(O)(C₁-C₁₀alkyl)-ONO₂, -O(C₁-C₁₀alkyl)-ONO₂.

17. A compound and the enantiomers, diastereoisomers and pharmaceutically acceptable salts thereof according to claim 16 wherein Y is a straight or branched C₁-C₁₀ alkylene.

18. A compound and the enantiomers, diastereoisomers and pharmaceutically acceptable salts thereof according to claim 15 wherein
Y is 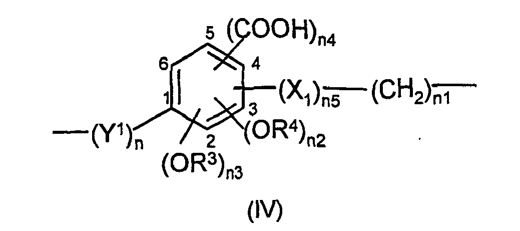 wherein
n is an integer from 0 to 20,
n1 is an integer from 1 to 20,
n2, n3, n4 and n5 are integers equal or different from each other, equal to 0 or 1;
R³ and R⁴ are independently selected from H or CH₃;
Y¹ is -CH₂- or -(CH₂)ₙₐ-CH=CH- wherein na is an integer from 0 to 20;
X₁ is -WC(O)- or -C(O)W-, wherein W is oxygen, sulfur or NH.

19. A compound and the enantiomers, diastereoisomers and pharmaceutically acceptable salts thereof according to claim 18 wherein
n2, n3, n4, n5 are equal to 0,
n1 is 1,
n is an integer from 0 to 10,
Y¹ is CH₂.

20. A compound and the enantiomers, diastereoisomers and pharmaceutically acceptable salts thereof according to claim 1 wherein Z and Z₁ are -C(O)-.

21. A compound and the enantiomers, diastereoisomers and pharmaceutically acceptable salts thereof according to claim 20 wherein
Y is a straight or branched C₁-C₂₀ alkylene being optionally substituted with one or more of the substituents selected from the group consisting of halogen atoms, hydroxy, -ONO₂ or T, wherein T is -OC(O)(C₁-C₁₀alkyl)-ONO₂, -O(C₁-C₁₀alkyl)-ONO₂.

22. A compound and the enantiomers, diastereoisomers and pharmaceutically acceptable salts thereof according to claim 21 wherein Y is a straight or branched C₁-C₁₀ alkylene.

23. A compound and the enantiomers, diastereoisomers and pharmaceutically acceptable salts thereof according to claim 20 wherein
Y is 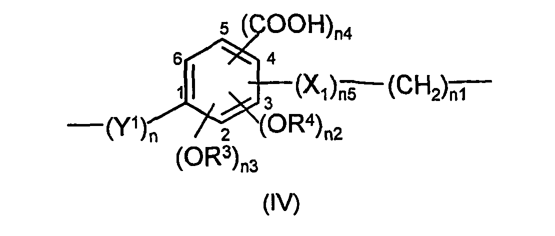 wherein
n is an integer from 0 to 20,
n1 is an integer from 1 to 20,
n2, n3, n4 and n5 are integers equal or different from each other, equal to 0 or 1,
R³ and R⁴ are independently selected from H or CH₃;
Y¹ is -CH₂- or-(CH₂)ₙₐ-CH=CH- wherein na is an integer from 0 to 20;
X₁ is WC(O)- or -C(O)W-, wherein W is oxygen, sulfur or NH.

24. A compound and the enantiomers, diastereoisomers and pharmaceutically acceptable salts thereof according to claim 23 wherein
n2, n3, n4, n5 are equal to 0,
n1 is 1,
n is an integer from 0 to 10,
Y¹ is CH₂.

25. A compound and the enantiomers, diastereoisomers and pharmaceutically acceptable salts thereof according to claim 23 wherein
n, n2, n5 are 1,
n3 and n4 are equal to 0,
n1 is an integer from 1 to 10,
Y¹ is -(CH₂)ₙₐ-CH=CH- wherein na is 0,
X₁ is WC(O)- wherein W is oxygen and X₁ is bound to the phenyl ring through the [C]₄, R⁴ is CH₃ and the group (OR⁴) is bound to the phenyl ring through the [C]₃.

26. A compound and the enantiomers, diastereoisomers and pharmaceutically acceptable salts thereof according to claim 20 wherein
Y is 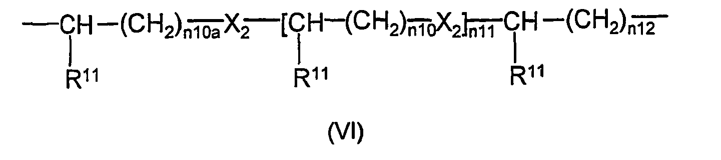 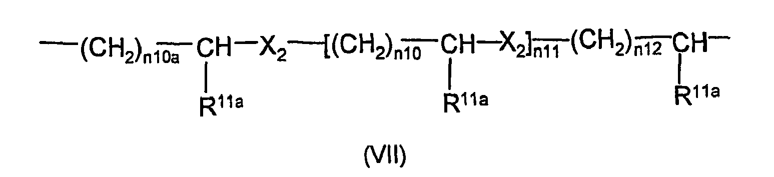 wherein
X₂ is O or S,
n10a, n10 and n12 are integers independently selected from 0 to 20;
n11 is an integer from 0 to 6;
R¹¹ is H, CH₃ or a nitrooxy group;
R^{11a} is CH₃ or a nitrooxy group.

27. A compound and the enantiomers, diastereoisomers and pharmaceutically acceptable salts thereof according to claim 26 wherein
Y is 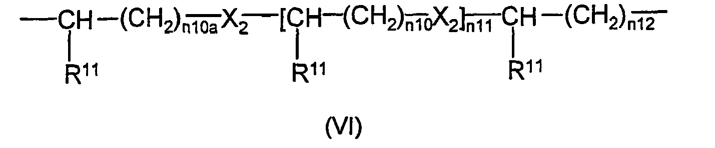 wherein
X₂ is O or S,
n10a and n11 are 0,
n12 is 1,
R¹¹ is H;
wherein the -ONO₂ group is bound to the -(CH₂)ₙ₁₂- group.

28. A compound and the enantiomers, diastereoisomers and pharmaceutically acceptable salts thereof according to claim 20 wherein
Y is 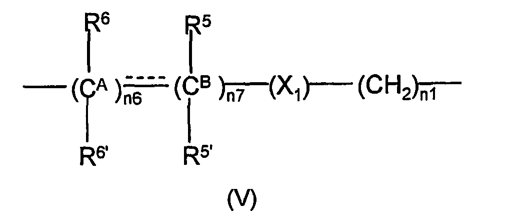 wherein:
n1 is an integer from 1 to 20;
X₁ is -WC(O)- or a -C(O)W-, wherein W is oxygen, sulfur or NH.
n6 is an integer from 1 to 20,
n7 is an integer from 0 to 20,
R⁵ and R^{5'} R⁶ and R^{6'} are independently selected from the group consisting of:
H, CH₃, OH, NH₂, NHCOCH₃, COOH, CH₂SH and C(CH₃)₂SH;
when the bond between the C^{A} and C^{B} carbons is a double bond R⁵ and R⁶ or R⁶' and
R^{5'} are absent.

29. A compound and the enantiomers, diastereoisomers and pharmaceutically acceptable salts thereof according to claim 28 wherein
n1 is an integer from 1 to 10,
n6 and n7 are 1;
X₁ is WC(O)- wherein W is sulfur,
R⁵, R^{5'} and R^{6'} are H,
R⁶ is NHCOCH₃,
with the proviso that the -ONO₂ group is bound to the -(CH₂)ₙ₁- group.

30. A compound and the enantiomers, diastereoisomers and pharmaceutically acceptable salts thereof according to claim 1 wherein s is 1, Z is H and Z₁ are -C(O)-.

31. A compound and the enantiomers, diastereoisomers and pharmaceutically acceptable salts thereof according to claim 30 wherein
Y is a straight or branched C₁-C₂₀ alkylene being optionally substituted with one or more of the substituents selected from the group consisting of halogen atoms, hydroxy, -ONO₂ or T, wherein T is -OC(O)(C₁-C₁₀alkyl)-ONO₂, -O(C₁-C₁₀alkyl)-ONO₂.

32. A compound and the enantiomers, diastereoisomers and pharmaceutically acceptable salts thereof according to claim 31 wherein Y is a straight or branched C₁-C₁₀ alkylene.

33. A compound and the enantiomers, diastereoisomers and pharmaceutically acceptable salts thereof according to claim 30 wherein
Y is 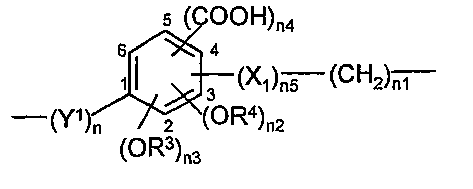 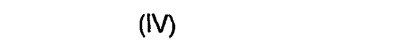 wherein
n is an integer from 0 to 20,
n1 is an integer from 1 to 20;
n2, n3, n4 and n5 are integers equal or different from each other, equal to 0 or 1;
R³ and R⁴ are independently selected from H or CH₃;
Y¹ is -CH₂- or -(CH₂)ₙₐ-CH=CH- wherein na is an integer from 0 to 20;
X₁ is -WC(O)- or -C(O)W-, wherein W is oxygen, sulfur or NH.

34. A compound and the enantiomers, diastereoisomers and pharmaceutically acceptable salts thereof according to claim 33 wherein
n2, n3, n4, n5 are equal to 0,
n1 is 1,
n is an integer from 0 to 10
Y¹ is CH₂.

35. A compound and the enantiomers, diastereoisomers and pharmaceutically acceptable salts thereof according to claim 33 wherein
n, n2, n5 are 1,
n3 and n4 are equal to 0,
n1 is an integer from 1 to 10,
Y¹ is -(CH₂)ₙₐ-CH=CH- wherein na is 0,
X₁ is WC(O)-, wherein W is oxygen and X₁ is bound to the phenyl ring through the
[C]₄,
R⁴ is CH₃ and the group (OR⁴) is bound to the phenyl ring through the [C]₃.

36. A compound and the enantiomers, diastereoisomers and pharmaceutically acceptable salts thereof according to claim 30 wherein
Y is 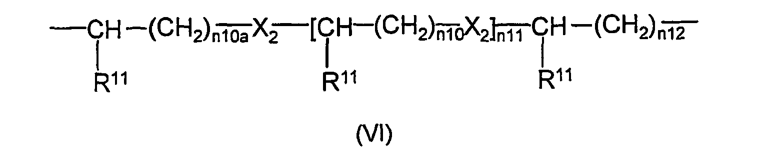 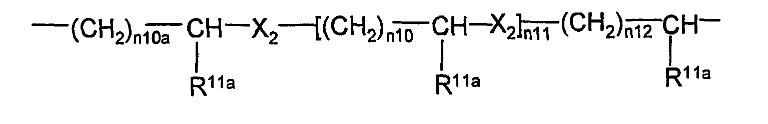 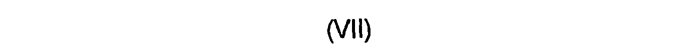 wherein
X₂ is O or S,
n10a, n10 and n12 are integers independently selected from 0 to 20;
n11 is an integer from 0 to 6;
R¹¹ is H, CH₃ or a nitrooxy group;
R^{11a} is CH₃ or a nitrooxy group.

37. A compound and the enantiomers, diastereoisomers and pharmaceutically acceptable salts thereof according to claim 36 wherein
Y is 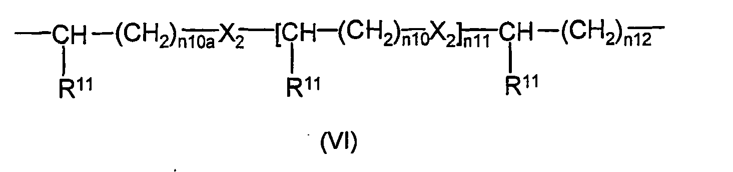 wherein
X₂ is O or S,
n10a and n11 are 0,
n12 is 1,
R¹¹ is H,
wherein the -ONO₂ group is bound to the -(CH₂)ₙ₁₂- group.

38. A compound and the enantiomers, diastereoisomers and pharmaceutically acceptable salts thereof according to claim 30 wherein
Y is 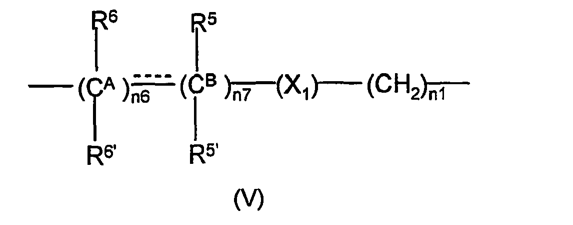 wherein:
n1 is an integer from 1 to 20;
X₁ is WC(O)- or a -C(O)W-, wherein W is oxygen, sulfur or NH.
n6 is an integer from 1 to 20,
n7 is an integer from 0 to 20,
R⁵ and R^{5'} R⁶ and R^{6'} are independently selected from the group consisting of: H, CH₃, OH, NH₂, NHCOCH₃, COOH, CH₂SH and C(CH₃)₂SH;
when the bond between the C^{A} and C^{B} carbons is a double bond R⁵ and R⁶ or R⁶' and
R^{5'} are absent.

39. A compound and the enantiomers, diastereoisomers and pharmaceutically acceptable salts thereof according to claim 38 wherein
n1 is an integer from 1 to 10,
n6 and n7 are 1;
X₁ is -WC(O)- wherein W is sulfur;
R⁵, R^{5'} and R^{6'} are H, R⁶ is NHCOCH₃;
with the proviso that the -ONO₂ group is bound to the -(CH₂)ₙ₁-.

40. Compounds and the enantiomers, diastereoisomers and pharmaceutically acceptable salts thereof according to claim 3 and claim 4 wherein the compounds are:

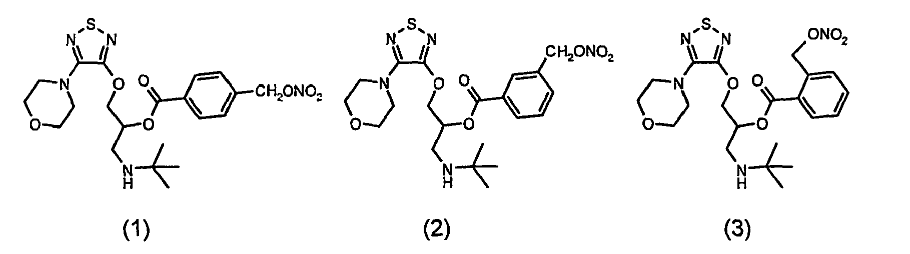

41. Compounds and the enantiomers, diastereoisomers and pharmaceutically acceptable salts thereof according to claim 20 and claim 24 wherein the compounds are:

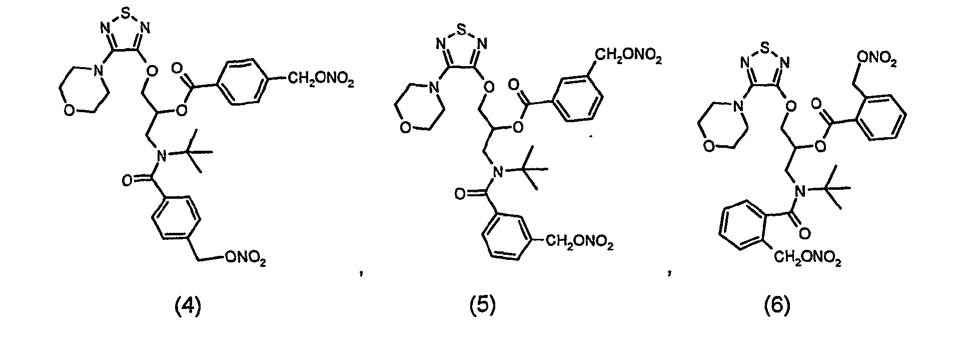

42. Compounds and the enantiomers, diastereoisomers and pharmaceutically acceptable salts thereof according to claim 30 and claim 34 wherein the compounds are:

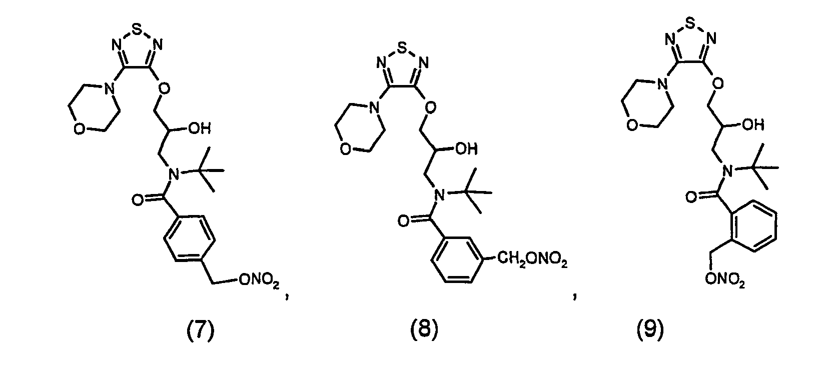

43. A compound and the enantiomers, diastereoisomers and pharmaceutically acceptable salts according to claim 1 and claim 4 which is 4-(Nitrooxymethyl)benzoic acid (S)-1-[(1,1-dimethylethyl)amino]-3-[[4-(4-morpholinyl)-1,2,5-thiadiazol-3-yl]oxy]-2-propanoate maleate salt.

44. A compound and the enantiomers, diastereoisomers and pharmaceutically acceptable salts according to claim 1 and claim 24, which is 4-(Nitrooxymethyl)benzoic acid (S)-1-[(1,1-dimethylethyl)[(4-nitrooxymethyl)benzoyl] amino]-3-[[4-(4-morpholinyl)-1,2,5-thiadiazol-3-yl]oxy]-2-propanoate.

45. A compound and the enantiomers, diastereoisomers and pharmaceutically acceptable salts according to claim 1 and claim 34 which is (S)-1-[(1,1-dimethylethyl)[(4-nitrooxymethyl)benzoyl]amino]-3-[[4-(4-morpholinyl)-1,2,5-thiadiazol-3-yl]oxy]-2-propanol.

46. A compound of formula (I) and/or the enantiomers, diastereoisomers and pharmaceutically acceptable salts thereof as defined in any of claims 1 to 45, for use as medicament.

47. Use of a compound of formula (I) and/or the enantiomers, diastereoisomers and pharmaceutically acceptable salts thereof as defined in any of claims 1 to 45 for preparing a drug that can be employed in the treatment or prophylaxis of hypertension, cardiovascular and vascular diseases.

48. Use of a compound of formula (I) and/or the enantiomers, diastereoisomers and pharmaceutically acceptable salts thereof as defined in any of claims 1 to 45 for preparing a drug that can be employed in the treatment of glaucoma and of elevated intraocular pressure.

49. A pharmaceutical composition comprising a compound of formula (I) and/or the enantiomers, diastereoisomers and pharmaceutically acceptable salts thereof as defined in any of claims 1 to 45 and a pharmaceutical acceptable carrier.

## Patentansprüche

1. Verbindung der allgemeinen Formel (I) A-(Y-ONO₂)ₛ und den Enantiomeren, Diastereomeren und pharmazeutisch verträglichen Salzen davon, wobei s eine ganze Zahl von 1 oder 2 ist;
A aus den folgenden β-adrenergen Blockergruppen der Formel (II) ausgewählt ist: 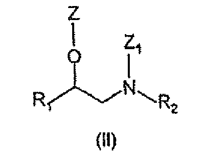 wobei
R₁ ausgewählt ist aus der Gruppe bestehend aus: 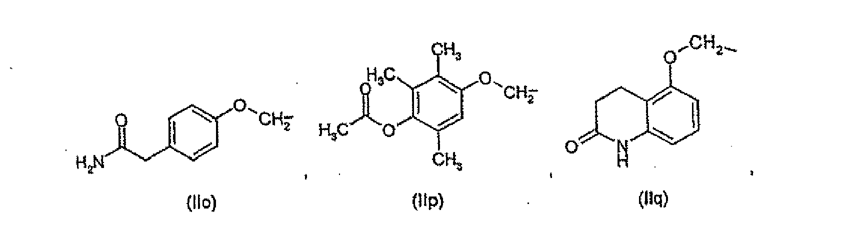 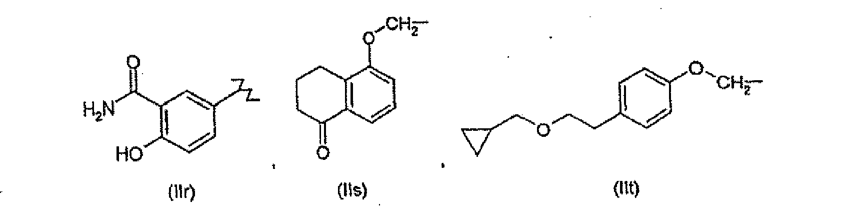 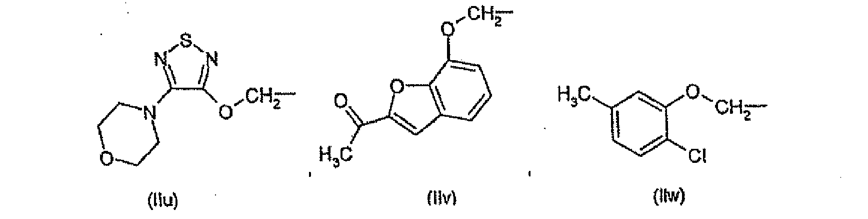 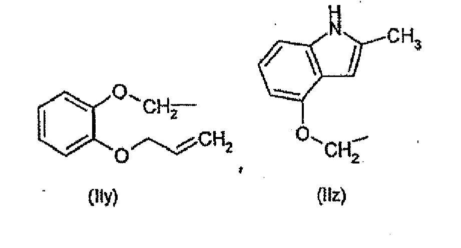 R₂ ausgewählt ist aus der Gruppe bestehend aus: -CH(CH₃)₂, -C(CH₃)₃ oder 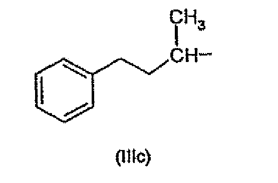 falls das Radikal R₁ aus den Formeln (IIo), (IIp), (IIt), (IIu), (IIv), (IIy) oder (IIz) ausgewählt wird ist R₂ -CH(CH₃)₂;
falls das Radikal R₁ aus den Formeln (IIq), (IIs) oder (IIw) ausgewählt wurde ist R₂
-C(CH₃)₃;
falls das Radikal R₁ (IIr) ist, ist R₂ (IIIc);
Z H ist, oder eine Gruppe, die fähig ist, Y zu blinden ausgewählt aus der Gruppe bestehend aus: -C(O)-, -C(O)O- oder 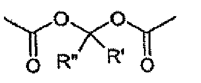 wobei R' und R" identisch oder verschieden sind und H oder ein verzweigtes oder unverzweigtes C₁-C₄ Alkyl sind;
Z₁ H ist oder eine -C(O)-Gruppe, die in der Lage ist, Y zu binden;
mit der Maßgabe, dass, falls s der Formel (I) 1 ist, Z oder Z₁ H sind;
Y ein bivalentes Radikal ist, das die folgende Bedeutung hat:
a) Verzweigtes oder unverzweigtes C₁-C₂₀ Alkylen, das optional mit einem oder mehreren Substituenten aus gewählt aus der Gruppe bestehend aus Halogenatomen, Hydroxy, -ONO₂ oder T substituiert ist, wobei T-OC(O)(C₁-C₁₀Alkyl)-ONO₂, -O(C₁-C₁₀ Alkyl)-ONO₂;
b) Cycloalken mit 5 bis 7 Kohlenstoffatomen im Cykloalkenring, wobei der Ring optional mit Seitenketten T₁ substituiert sind, wobei T₁ ein verzweigtes oder unverzweigtes Alkyl ist, mit 1 bis 10 Kohlenstoffatomen;
c) 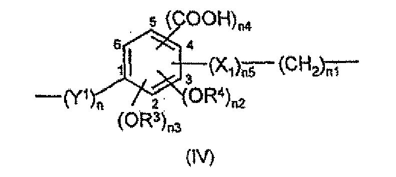 wobei:
n eine ganze Zahl von 0 bis 20 ist und n1 eine ganze Zahl von 1 bis 20 ist;
n2, n3, n4 und n5 gleiche oder unterschiedliche ganze Zahlen sind, die gleich 0 oder 1 sind;
R³ und R⁴ unabhängig voneinander ausgewählt sind aus H oder CH₃;
Y¹ -CH₂- oder -(CH₂)ₙₐ-CHK=CH- ist, wobei na eine ganze Zahl von 0 bis 20 ist;
X¹ -WC(O)- oder-C(O)W- ist, wobei W Sauerstoff, Schwefel, oder NH ist;
mit der Maßgabe, dass:
- Wenn s der Formel (I) 1 ist, Z -(CO)- ist und wenn in Formel (IV) des bivalenten Radikals Y n2, n3, n4, n5 gleich 0 sind, n 0 ist und n1 1 ist;
- Wenn s in Formel (I) 1 ist, Z -(CO)- ist und wenn in Formel (IV) des bivalenten Radikals Y n2, n3, n5 gleich 0 sind und n4 1 ist, n und n1 ungleich 1 sind;
d) 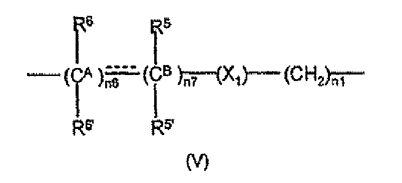 wobei:
n1 eine ganze Zahl von 1 bis 20 ist;
X₁ -WC(O)- oder -C(O)W- ist, wobei W Sauerstoff, Schwefel, oder NH ist;
n6 eine ganze Zahl von 1 bis 20 ist,
n7 eine ganze Zahl von 1 bis 20 ist,
R⁵ und R^{5'}, R⁶ und R^{6'} unabhängig voneinander ausgewählt sind aus der Gruppe bestehend aus H, CH₃, OH, NH₂, NHCOCH₃, COOH, CH₂SH und C(CH₃)₂SH; falls die Bindung zwischen dem C^{A} und dem C^{B} Kohlenstoff eine Doppelbindung ist, sind R⁵ und R⁶ oder R^{6'} und R^{5'} abwesend;
mit der Maßgabe, dass falls Y aus den unter c) - d) aufgeführten bivalenten Radikalen ausgewählt ist, die -ONO₂ Gruppe an eine -(CH₂)ₙ₁ Gruppe gebunden ist;
mit der Maßgabe, dass wenn s der Formel (I) 1 ist und Z-(CO)- ist, das bivalente Radikal nicht die Bedeutung wie unter a), b) und d) hat;
e) 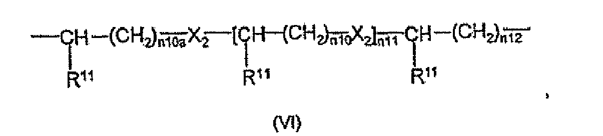 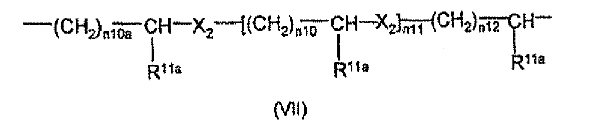 wobei X₂ O oder S ist,
n10a, n10 und n12 ganze Zahlen sind, die unabhängig ausgewählt sind aus 0 bis 20,
n11 eine ganze Zahl von 0 bis 6 ist,
R¹¹ H, CH₃ oder eine Nitrooxygruppe ist;
mit der Maßgabe, dass wenn s in Formel (I) 1 ist, Z in Formel (II) -(CO)- ist, in Formel (VI) des bivalenten Radikals Y n10a, n10, n12 gleich 1 sind, X kein Sauerstoffatom sein kann
f) 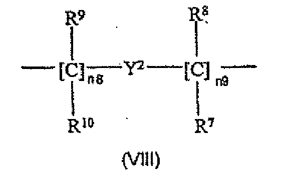
wobei:
n8 eine ganze Zahl von 0 bis 10 ist;
n9 eine ganze Zahl von 1 bis 10 ist;
R⁹, R¹⁰, R⁸, R⁷ identisch oder unterschiedlich sind und H oder ein verzweigtes oder
unverzweigtes C₁-C₄ Alkyl sind;
wobei die -ONO₂ Gruppe an 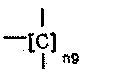 gebunden ist wobei n9 wie oben definiert ist;
Y² ein heterozyklischer, gesättigter, ungesättigter oder aromatischer 5er oder 6er Ring ist, der ein oder mehrere Heteroatom(e) aus gewählt aus Stickstoff, Sauerstoff, Schwefel enthält und ausgewählt ist aus der Gruppe bestehend aus:

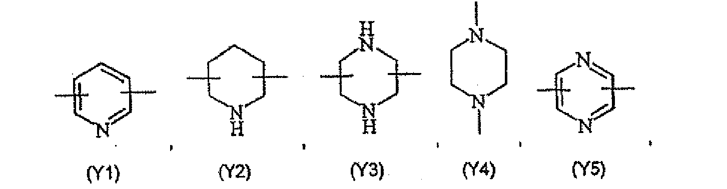

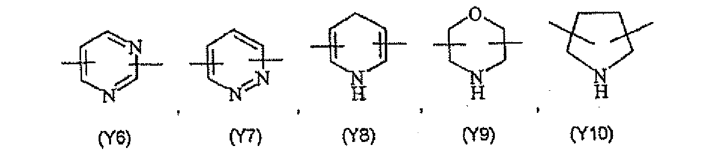

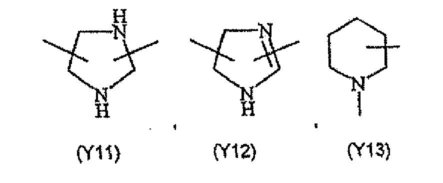

2. Verbindung, die Enantiomere, Diastereomere und pharmazeutisch verträglichen Salze davon gemäß Anspruch 1, wobei s gleich 1 ist und Z₁ gleich H ist.

3. Verbindung, die Enantiomere, Diastereomere und pharmazeutisch verträglichen Salze davon gemäß Anspruch 2, wobei Z -C(O)- ist.

4. Verbindung, die Enantiomere, Diastereomere und pharmazeutisch verträglichen Salze davon gemäß Anspruch 3, wobei Y 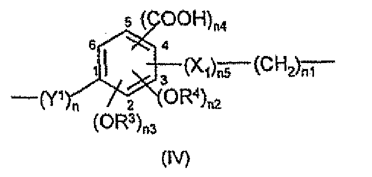 ist, wobei
n, n2, n3, n4 und n5 0 sind
n1 eine ganze Zahl von 1 ist.

5. Verbindung, die Enantiomere, Diastereomere und pharmazeutisch verträglichen Salze davon gemäß Anspruch 3, wobei Y 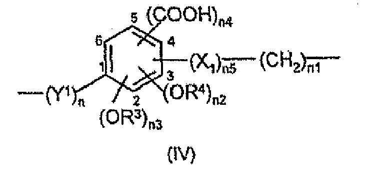 ist, wobei
n, n2, n5 1 sind,
n3 und n4 gleich 0 sind,
n1 eine ganze Zahl von 1 bis 10 ist,
Y¹ -(CH₂)ₙₐ-CH=CH- ist, wobei na 0 ist,
X₁ -WC(O)- ist, wobei W Sauerstoff ist und X₁ über das [C]₄ an den Phenylring gebunden ist,
R⁴ CH₃ ist und die (OR⁴) Gruppe über das [C]₃ an den Phenylring gebunden ist.

6. Verbindung, die Enantiomere, Diastereomere und pharmazeutisch verträglichen Salze davon gemäß Anspruch 3, wobei Y 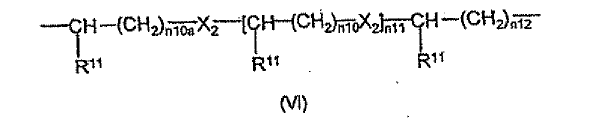 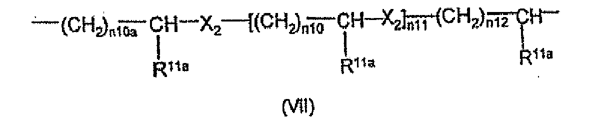 ist, wobei
X₂ O oder S ist,
n10a, n10 und n12 ganze Zahlen sind, die unabhängig voneinander ausgewählt sind von 2 bis 20;
n11 eine ganze Zahl von 0 bis 6 ist;
R¹¹ H, CH oder eine Nitrooxygruppe ist;
R^{11a} CH₃ oder eine Nitrooxygruppe ist.

7. Verbindung, die Enantiomere, Diastereomere und pharmazeutisch verträglichen Salze davon gemäß Anspruch 2, wobei Z -C(O)O- ist.

8. Verbindung, die Enantiomere, Diastereomere und pharmazeutisch verträglichen Salze davon gemäß Anspruch 7, wobei
Y ein verzweigtes oder unverzweigtes C₁-C₂₀ Alkylen ist, das optional mit einem oder mehreren der Substituenten substituiert ist, die ausgewählt sind aus der Gruppe bestehend aus Halogenatomen, Hydroxy, -ONO₂ oder T, wobei T -OC(O)(C₁-C₁₀ Alkyl)-ONO₂, -O(C₁-C₁₀Alkyl)-ONO₂ ist.

9. Verbindung, die Enantiomere, Diastereomere und pharmazeutisch verträglichen Salze davon gemäß Anspruch 8, wobei Y ein verzweigtes oder unverzweigtes C₁-C₁₀ Alkylen ist.

10. Verbindung, die Enantiomere, Diastereomere und pharmazeutisch verträglichen Salze davon gemäß Anspruch 7, wobei Y 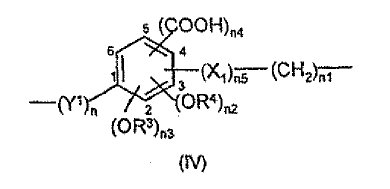 ist, wobei
n eine ganze Zahl von 0 bis 20 ist,
n1 eine ganze Zahl von 1 bis 20 ist;
n2, n3, n4 und n5 identische oder voneinander unterschiedliche ganze Zahlen sind, sie 0 oder 1 sind;
R³ und R⁴ unabhängig voneinander ausgewählt sind aus H oder CH₃;
Y¹ -CH₂- oder -(CH₂)ₙₐ-CH=CH- ist, wobei na eine ganze Zahl von 0 bis 20 ist;
X₁ -WC(O)- oder -C(O)W- ist, wobei W Sauerstoff, Schwefel oder NH ist.

11. Verbindung, die Enantiomere, Diastereomere und pharmazeutisch verträglichen Salze davon gemäß Anspruch 10, wobei
n2, n3, n4, n5 gleich 0 sind,
n1 gleich 1 ist,
n eine ganze Zahl von 0 bis 10 ist,
Y¹ CH₂ ist.

12. Verbindung, die Enantiomere, Diastereomere und pharmazeutisch verträglichen Salze davon gemäß Anspruch 7, wobei Y 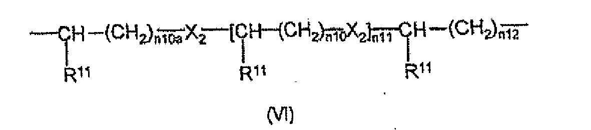 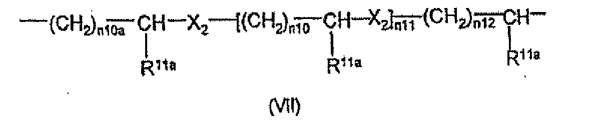 ist, wobei
X₂ O oder S ist,
n10a, n10 und n12 unabhängig voneinander ausgewählte ganze Zahlen von 0 bis 20 sind; n11 eine ganze Zahl von 0 bis 6 ist;
R¹¹ H, CH₃ oder eine Nitrooxygruppe ist;
R^{11a} CH₃ oder eine Nitrooxygruppe ist.

13. Verbindung, die Enantiomere, Diastereomer und pharmazeutisch verträglichen Salze davon gemäß Anspruch 12, wobei Y 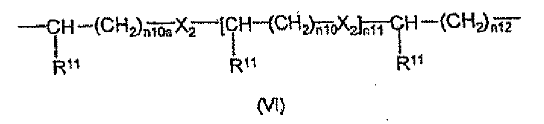 ist, wobei
X₂ O oder S ist,
n10a und n11 0 sind,
n12 1 ast und
R¹¹ H ist;
wobei die -ONO₂ Gruppe an die -(CH₂)ₙ₁₂₋ Gruppe gebunden ist.

14. Verbindung, die Enantiomere, Diastereomere und pharmazeutisch verträglichen Salze
davon gemäß Anspruch 7, wobei Y 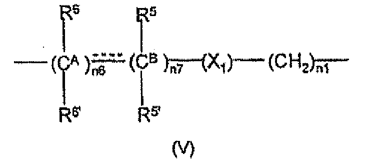 ist, wobei
n1 eine ganze Zahl von 1 bis 20 ist,
X₁-WC(O)- oder -C(O)W- ist, wobei W Sauerstoff, Schwefel oder NH ist,
n6 eine ganze Zahl von 1 bis 20 ist,
n76 eine ganze Zahl von 0 bis 20 ist,
R⁵ und R^{5'}, R⁶ und R^{6'} unabhängig voneinander ausgewählt sind aus der Gruppe bestehend aus: H, CH₃, OH, NH₂, NHCOCH₃, COOH, CH₂SH und C(CH₃)₂SH; falls die Bindung zwischen den C^{A} und C^{B} Kohlenstoffatomen eine Doppelbindung ist, sind R⁵ und R⁶ oder R^{5'} und R^{6'} abwesend.

15. Verbindung, die Enantiomere, Diastereomere und pharmazeutisch verträglichen Salze davon gemäß Anspruch 2, wobei Z 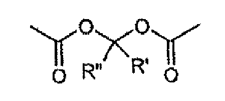 ist.

16. Verbindung, die Enantiomere, Diastereomere und pharmazeutisch verträglichen Salze davon gemäß Anspruch 15, wobei
Y ein verzweigtes oder unverzweigtes C₁-C₂₀ Alkylen ist, das optional mit einem oder mehreren Substituenten substituiert ist, ausgewählt aus der Gruppe bestehend aus Halogenatomen, Hydroxy, -ONO₂ oder T, wobei T -OC(O)(C₁-C₁₀ Alkyl)-ONO₂, - O(C₁-C₁₀Alkyl)-ONO₂, ist.

17. Verbindung, die Enantiomere, Diastereomere und pharmazeutisch verträglichen Salze davon gemäß Anspruch 16, wobei Y ein unverzweigte oder verzweigtes C₁-C₁₀ Alkylen ist.

18. Verbindung, die Enantiomere, Diastereomere und pharmazeutisch verträglichen Salze davon gemäß Anspruch 15, wobei Y 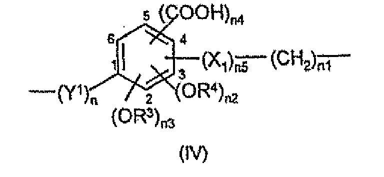 ist, wobei
n eine ganze Zahl von 0 bis 20 ist,
n1 eine ganze Zahl von 1 bis 20 ist,
n2, n3, n4 und n5 identische oder unterschiedlich von einander ganze Zahlen von 0 oder 1 sind;
R³ und R⁴ unabhängig voneinander aus H oder CH₃ ausgewählt sind;
Y¹ -CH₂- oder -(CH₂)ₙₐ-CH=CH- ist, wobei na eine ganze Zahl von 0 bis 20 ist;
X₁ -WC(O)- oder -C(O)W- ist, wobei W Sauerstoff, Schwefel oder NH ist.

19. Verbindung, die Enantiomere, Diastereomere und pharmazeutisch verträglichen Salze davon gemäß Anspruch 18, wobei
n2, n3, n4, n5 gleich 0 sind,
n1 1 ist,
n eine ganze Zahl von 0 bis 10 ist,
Y¹ CH₂ ist.

20. Verbindung, die Enantiomere, Diastereomere und pharmazeutisch verträglichen Salze davon gemäß Anspruch 1, wobei Z und Z₁ -C(O)- sind.

21. Verbindung, die Enantiomere, Diastereomere und pharmazeutisch verträglichen Salze davon gemäß Anspruch 20, wobei
Y ein verzweigtes oder unverzweigtes C₁-C₂₀ Alkylen ist, das optional mit einem oder mehreren Substituenten substituiert ist, ausgewählt aus der Gruppe bestehend aus Halogenatomen, Hydroxy, -ONO₂ oder T, wobei T -OC(O)(C₁-C₁₀Akyl)-ONO₂, - O(C₁-C₁₀Alkyl)-ONO₂ ist.

22. Verbindung, die Enantiomere, Diastereomere und pharmazeutisch verträglichen Salze davon gemäß Anspruch 21, wobei Y ein verzweigtes oder unverzweigtes C₁-C₁₀ Alkylen ist.

23. Verbindung, die Enantiomere, Diastereomere und pharmazeutisch verträglichen Salze davon gemäß Anspruch 20, wobei Y 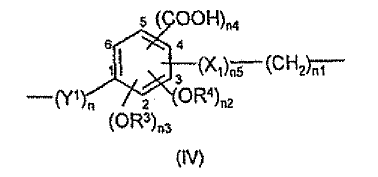 ist, wobei
n eine ganze Zahl von 0 bis 20 ist,
n1 eine ganze Zahl von 1 bis 20 ist,
n2, n3, n4, n5 gleiche oder voneinander unterschiedliche ganze Zahlen sind, die 0 oder 1 sind,
R³ und R⁴ unabhängig voneinander aus H und CH₃ ausgewählt sind;
Y¹ -CH₂- oder -(CH₂)ₙₐ-CH=CH- ist, wobei na eine ganze Zahl von 0 bis 20 ist;
X₁ -WC(O)- oder -C(O)W- ist, wobei W Sauerstoff, Schwefel oder NH ist.

24. Verbindung, die Enantiomere, Diastereomere und pharmazeutisch verträglichen Salze davon gemäß Anspruch 23, wobei
n2, n3, n4, n5 gleich 0 sind,
n1 gleich 1 ist,
n eine ganze Zahl von 0 bis 10 ist,
Y¹ CH₂ ist.

25. Verbindung, die Enantiomere, Diastereomere und pharmazeutisch verträglichen Salze davon gemäß Anspruch 23, wobei
n, n2, n5 1 sind,
n3 und n4 gleich 0 sind,
n1 eine ganze Zahl von 1 bis 10 ist,
Y¹ -(CH₂)ₙₐ-CH=CH- ist, wobei na 0 ist,
X₁ -WC(O)- ist, wobei W Sauerstoff ist und X₁ über das [C]₄ an den Phenylring gebunden ist, R⁴ CH₃ ist und die Gruppe (OR⁴) über das [C]₃ an den Phenylring gebunden ist.

26. Verbindung, die Enantiomere, Diastereomere und pharmazeutisch verträglichen Salze davon gemäß Anspruch 20, wobei Y 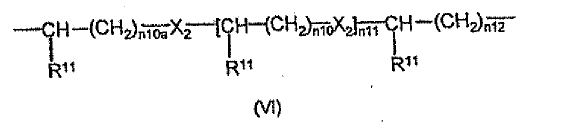 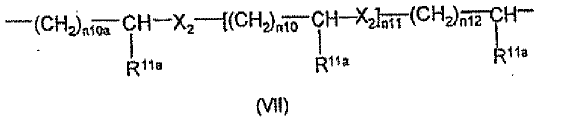 ist,
wobei
X₂ O oder S ist,
n10a, n10 und n12 ganze Zahlen sind, die unabhängig voneinander ausgewählt sind von 0 bis 20;
n11 eine ganze Zahl von 0 bis 6 ist;
R¹¹ H, CH₃ oder eine Nitrooxygruppe ist;
R^{11a} CH₃ oder eine Nitrooxygruppe ist.

27. Verbindung, die Enantiomere, Diastereomer und pharmazeutisch verträglichen Salze davon gemäß Anspruch 26, wobei Y 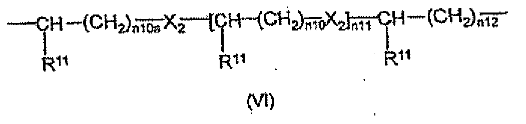 ist,
wobei
X₂ O oder S ist,
n10a und n11 0 sind,
n12 1 ist,
R¹¹ H ist;
wobei die -ONO₂ Gruppe an die -(CH₂)ₙ₁₂ Gruppe gebunden ist.

28. Verbindung, die Enantiomere, Diastereomer und pharmazeutisch verträglichen Salze davon gemäß Anspruch 20, wobei Y 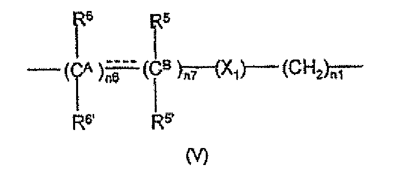 ist,
wobei:
n1 eine ganze Zahl von 1 bis 20 ist;
X₁ -WC(O)- oder ein -C(O)W- ist, wobei W Sauerstoff, Schwefel oder NH ist
n6 eine ganze Zahl von 1 bis 20 ist,
n7 eine ganze Zahl von 0 bis 20 ist,
R⁵ und R^{5'}, R⁶ und R^{6'} unabhängig voneinander aus der Gruppe bestehend aus: H, CH₃, OH, NH₂, NHCOCH₃, COOH, CH₂SH und C(CH₃)₂SH ausgewählt sind;
wenn die Bindung zwischen den C^{A} und C^{B} Kohlenstoffen eine Doppelbindung ist, sind R⁵ und R⁶ oder R^{6'} und R^{5'} abwesend.

29. Verbindung, die Enantiomere, Diastereomere und pharmazeutisch verträglichen Salze davon gemäß Anspruch 28, wobei
n1 eine ganze Zahl von 1 bis 10 ist,
n6 und n7 1 sind;
X₁ -WC(O)- ist, wobei W Schwefel ist,
R⁵, R^{5'} und R^{6'} H sind,
R⁶ NHCOCH₃ ist,
mit der Maßgabe, dass die -ONO₂ Gruppe an die -(CH₂)ₙ₁ Gruppe gebunden ist.

30. Verbindung, die Enandomere, Diastereomere und pharmazeutisch verträglichen Salze davon gemäß Anspruchs wobei s 1 ist, Z H ist und Z₁-C(O)- ist.

31. Verbindung, die Enantiomere, Diastereomere und pharmazeutisch verträglichen Salze davon gemäß Anspruch 30, wobei
Y ein verzweigtes oder unverzweigtes C₁-C₂₀ Alkylen ist, das optional mit einem oder mehreren der Substituenten substituiert ist, die ausgewählt sind aus der Gruppe bestehend aus Halogenatomen, Hydroxy, -ONO₂ oder T, wobei T-OC(O)(C₁-C₁₀ Alkyl)-ONO₂, -O(C₁-C₁₀ Alkyl)-ONO₂ ist.

32. Verbindung, die Enantiomere, Diastereomere und pharmazeutisch verträglichen Salze davon gemäß Anspruch 31, wobei Y ein verzweigtes oder unverzweigtes C₁-C₁₀ Alkylen ist.

33. Verbindung, die Enantiomere, Diastereomere und pharmazeutisch verträglichen Salze davon gemäß Anspruch 30, wobei Y 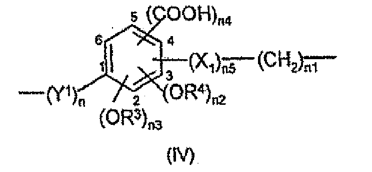 ist, wobei
n eine ganze Zahl von 0 bis 20 ist,
n1 eine ganze Zahl von 1 bis 20 ist;
n2, n3, n4 und n5 gleiche oder unterschiedliche ganze Zahlen sind, die gleich 0 oder 1 sind;
R³ und R⁴ unabhängig voneinander aus H oder CH₃ ausgewählt sind;
Y¹ -CH₂- oder -(CH₂)ₙₐ-CH=CH- ist, wobei na eine ganze Zahl von 0 bis 20 ist;
X₁ -W(O)- oder -C(O)W- ist, wobei W Sauerstoff, Schwefel oder NH ist.

34. Verbindung, die Enantiomere, Diastereomere und pharmazeutisch verträglichen Salze davon gemäß Anspruch 33, wobei
n2, n3, n4, n5 gleich 0 sind,
n1 1 ist,
n eine ganze Zahl von 0 bis 10 ist
Y¹ CH₂ ist.

35. Verbindung, die Enantiomere, Diastereomere und pharmazeutisch verträglichen Salze davon gemäß Anspruch 33, wobei
n, n2, n5 1 sind,
n3 und n4 gleich 0 sind,
n1 eine ganze Zahl von 1 bis 10 ist,
Y¹ -(CH₂)ₙₐ-CH=CH- ist, wobei na 0 ist,
X₁ -WC(O)- ist, wobei W Sauerstoff ist und X₁ über das [C]₄ an den Phenylring gebunden ist,
R⁴ CH₃ ist und die (OR⁴) Gruppe über das [C]₃ an den Phenylring gebunden ist.

36. Verbindung, die Enantiomere, Diastereomere und pharmazeutisch verträglichen Salze davon gemäß Anspruch 30, wobei Y 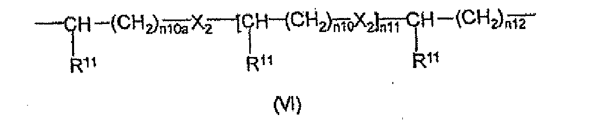 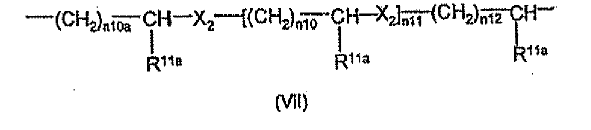 ist,
wobei
X₂ O oder S ist,
n10a, n10 und n12 ganze Zahlen sind, die unabhängig voneinander von 0 bis 20 ausgewählt sind;
n11 eine ganze Zahl von 0 bis 6 ist;
R¹¹ H, CH₃ oder eine Nitrooxygruppe ist;
R^{11a} CH₃ oder eine Nitrooxygruppe ist.

37. Verbindung, die Enantiomer, Diastereomere und pharmazeutisch verträglichen Salze davon gemäß Anspruch 36, wobei Y 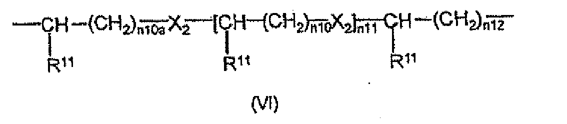 ist,
wobei
X₂ O oder S ist,
n10a und n11 0 sind,
n12 1 ist,
R¹¹ H ist;
wobei die -ONO₂ Gruppe an die -(CH₂)ₙ₁₂ Gruppe gebunden ist.

38. Verbindung, die Enantiomere, Diastereomere und pharmazeutisch verträglichen Salze davon gemäß Anspruch 30, wobei Y 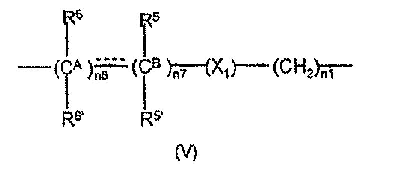 ist,
wobei:
n1 eine ganze Zahl von 1 bis 20 ist;
X₁ -WC(O)- oder ein -C(O)W- ist, wobei W Sauerstoff, Schwefel oder NH ist
n6 eine ganze Zahl von 1 bis 20 ist,
n7 eine ganze Zahl von 0 bis 20 ist,
R⁵ und R^{5'}, R³ und R^{6'} unabhängig voneinander aus der Gruppe bestehend aus: H, CH₃,
OH, NH₂, NHCOCH₃, COOH, CH₂SH und C(CH₃)₂SH ausgewählt sind;
wenn die Bindung zwischen den C^{A} und C^{B} Kohlenstoffen eine Doppelbindung ist, sind R⁵ und R⁶ oder R^{6'} und R^{5'} abwesend.

39. Verbindung, die Enantiomer, Diastereomere und pharmazeutisch verträglichen Salze davon gemäß Anspruch 38, wobei
n1 eine ganze Zahl von 1 bis 10 ist,
n6 und n7 1 sind;
X₁ -WC(O)- ist, wobei W Schwefel ist,
R⁵, R^{5'} und R^{6'} H sind, R⁶ NHCCOCH₃ ist;
mit der Maßgabe, dass die -ONO₂ Gruppe an die -(CH₂)ₙ₁ Gruppe gebunden ist.

40. Verbindung, die Enantiomere, Diastereomere und pharmazeutisch verträglichen Salze davon gemäß Anspruch 3 und Anspruch 4, wobei die Verwindungen 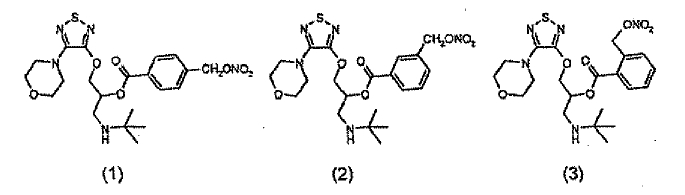 sind.

41. Verbindung, die Enantiomere, Diastereomere und pharmazeutisch verträglichen Salze davon gemäß Anspruch 20 und Anspruch 24, wobei die Verbindungen 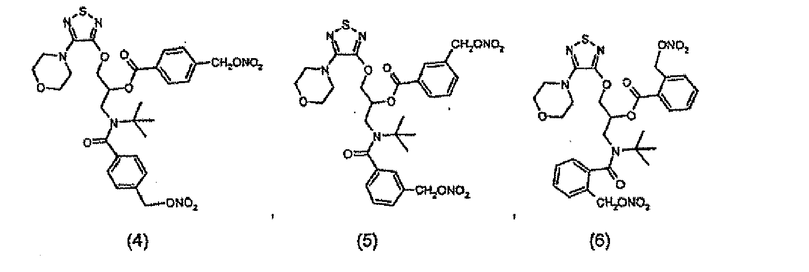 sind.

42. Verbindung, die Enantiomere, Diastereomere und pharmazeutisch verträglichen Salze davon gemäß Anspruch 30 und Anspruch 34, wobei die Verbindungen 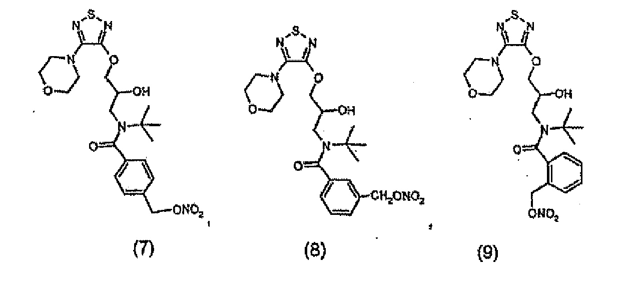 sind.

43. Verbindung, die Enantiomere, Diastereomere und pharmazeutisch verträglichen Salze davon gemäß Anspruch 1 und Anspruch 4, die 4-(Nitrooxymethyl) benzoesäure (S)-1-[(1,1-dimethylethyl) amino]-3-[[4-(4-morpholinyl)-2,2,5-thiadiazol-3-yl] oxy]-2-propanoatmaleatsalz ist.

44. Verbindung, die Enantiomere, Diastereomere und pharmazeutisch verträglichen Salze davon gemäß Anspruch 1 und Anspruch 24, die 4-(Nitrooxymethyl) benzoesäure (S)-1-[(1,1-dimethylethyl)[(4-nitrooxymethyl) benzoyl] amino]-3-[[4-(4-morpholinyl)-1,2,5-thiadiazol-3-yl] oxy]-2-propanoat ist.

45. Verbindung, die Enantiomere, Diastereomere und pharmazeutisch verträglichen Salze davon gemäß Anspruch 1 und Anspruch 34, die (S)-1-[(1,1-dimethylethyl)[(4-nitrooxymethyl) benzoyl] amino]-3-[[4-(4-morpholinyl)-1,2,5-thiadiazol-3-yl] oxy]-2-propanol ist.

46. Verbindung der Formel (I) und/oder die Enantiomere, Diastereomere und pharmazeutisch verträglichen Salze davon, wie in jeglichem der Ansprüche 1 bis 45 definiert zur Verwendung als Medikament.

47. Verwendung einer Verbindung der Formel (I) und/oder der Enantiomere, Diastereomere und pharmazeutisch verträglichen Salze davon, wie in jeglichem der Ansprüche 1 bis 45 definiert zur Herstellung eines Wirkstoffes, der für die Behandlung oder Prophylaxe von Hypertonie, kardiovaskulären und vaskulären Erkrankungen eingesetzt werden kann.

48. Verwendung einer Verbindung der Formel (I) und/oder der Enantiomere, Diastereomer und pharmazeutisch verträglichen Salze davon, wie in jeglichem der Ansprüche 1 bis 45 definiert zur Herstellung eines Wirkstoffs, der für die Behandlung eines Glaukoms und erhöhten intraokularen Druckes eingesetzt werden kann.

49. Pharmazeutische Zusammensetzung umfassend eine Verbindung nach Formel (I) und/oder der Enantiomere, Diastereomere und pharmazeutisch verträglichen Salze davon, wie in jeglichem der Ansprüche 1 bis 45 definiert und einen pharmazeutisch verträglichen Trägerstoff.

## Revendications

1. Composé répondant à la formule générale (I) A-(Y-ONO₂)ₛ ainsi que ses énantiomères, ses diastéréo-isomères et ses sels pharmaceutiquement acceptables, dans lequel
s représente un entier égal à 1 ou 2 ;
A est choisi parmi les résidus β-bloquants suivants répondant à la formule (II) : 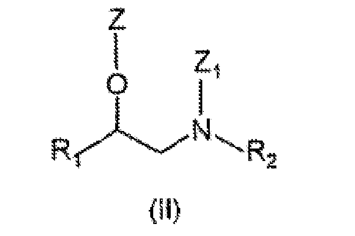 dans laquelle
R₁ est choisi parmi le groupe constitué par : 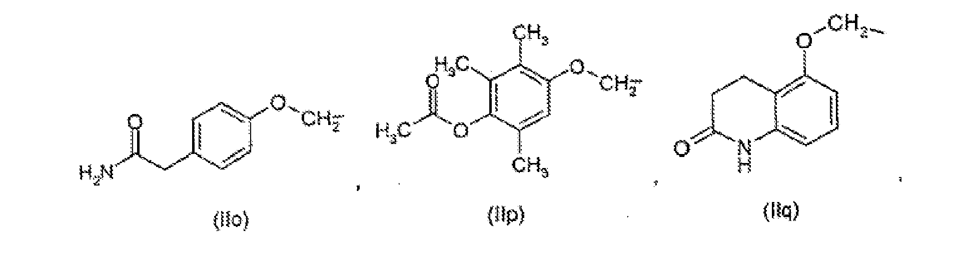 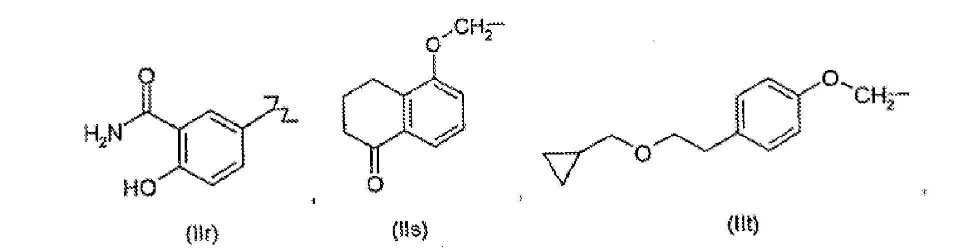 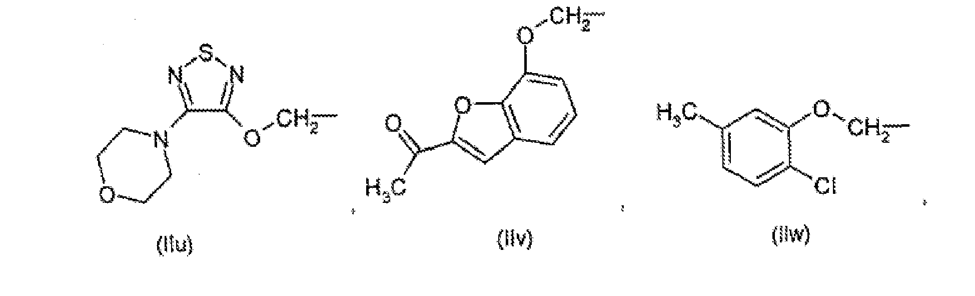 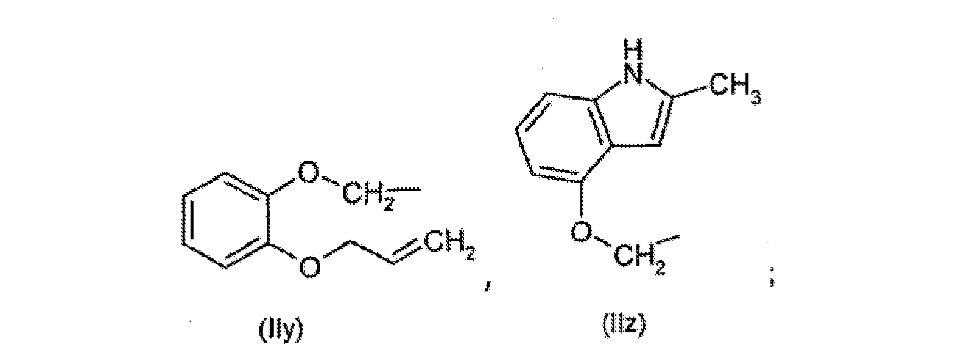 R₂ est choisi parmi le groupe constitué par : un groupe -CH(CH₃)₂, un groupe -C(CH₃)₃ ou un groupe 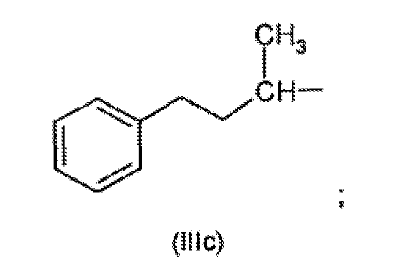 lorsque le radical R₁ est choisi parmi les formules (IIo), (IIp), (IIt), (IIu), (IIv), (IIy) ou (IIz), R₂ représente un groupe -CH(CH₃)₂ ;
lorsque le radical R₁ est choisi parmi les formules (IIq), (IIs) ou (IIw), R₂ représente un groupe -C(CH₃)₃ ;
lorsque le radical R₁ répond à la formule (IIr), R₂ répond à la formule (IIIc) ;
Z représente un atome d'hydrogène ou représente un groupe capable de se lier à Y, choisi parmi le groupe constitué par : un groupe -C(O)-, un groupe -C(O)O- ou un groupe : 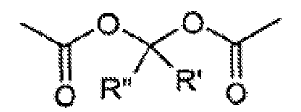 dans lequel R' et R" sont identiques ou différents, et représentent un atome d'hydrogène ou un groupe alkyle en C₁-C₄ à chaîne droite ou ramifiée ;
Z₁ représente un atome d'hydrogène ou un groupe -C(O)- capable de se lier à Y ;
avec cette réserve que, lorsque s répondant à la formule (I) est égal à 1, Z ou Z₁ représente un atome d'hydrogène ;
Y représente un radical divalent possédant la signification suivante :
a)
- un groupe alkylène en C₁-C₂₀ à chaîne droite ou ramifiée substitué de manière facultative par un ou plusieurs des substituants choisis parmi le groupe constitué par un atome d'halogène, un groupe hydroxyle, un groupe -ONO₂ ou un groupe T, T représentant un groupe -OC(O)(alkyl en C₁-C₁₀)-ONO₂, un groupe -O(alkyl en C₁-C₁₀)-ONO₂;
b)
- un groupe cycloalkylène contenant de 5 à 7 atomes de carbone dans le noyau cycloalkylène, le noyau étant substitué de manière facultative par des chaînes latérales T₁, T₁ représentant un groupe alkyle à chaîne droite ou ramifiée contenant de 1 à 10 atomes de carbone ;
c)
un groupe 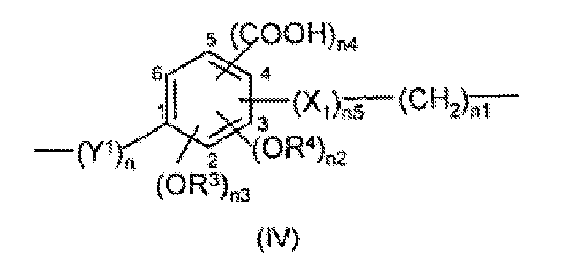 dans lequel
n représente un entier de 0 à 20 et n1 représente un entier de 1 à 20 ;
n2, n3, n4 et n5 représentent des entiers égaux l'un à l'autre ou différents l'un de l'autre, égaux à 0 ou 1 ;
R³ et R⁴ sont choisis, de manière indépendante, parmi un atome d'hydrogène ou un groupe CH₃ ;
Y¹ représente un groupe -CH₂- ou -(CH₂)ₙₐ-CH=CH- dans lequel na représente un entier de 0 à 20 ;
X₁ représente un groupe -WC(O)- ou un groupe -C(O)W- dans lequel W représente un atome d'oxygène, un atome de soufre ou un groupe NH ;
avec cette réserve que :
- lorsque s répondant à la formule (I) est égal à 1, Z représente un groupe -(CO)- et lorsque, dans la formule (IV) du radical divalent Y, n2, n3, n4, n5 sont égaux à 0, n est égal à 0 et n1 est égal à 1 ;
- lorsque s répondant à la formule (I) est égal à 1, Z représente un groupe -(CO)- et lorsque, dans la formule (IV) du radical divalent Y, n2, n3, n5 sont égaux à 0, n4 est égal à 1, n et n1 sont différents de 1 ;
d)
un groupe 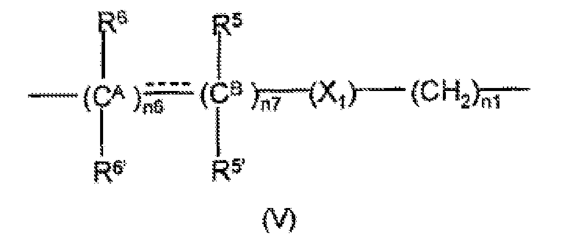 dans lequel :
n1 représente un entier de 1 à 20 ;
X¹ représente un groupe -WC(O)- ou un groupe -C(O)W- dans lequel W représente un atome d'oxygène, un atome de soufre ou un groupe NH ;
n6 représente un entier de 1 à 20 ;
n7 représente un entier de 0 à 20 ;
R⁵ et R^{5'}, R⁶ et R^{6'} sont choisis, indépendamment l'un de l'autre, parmi le groupe constitué par un atome d'hydrogène, un groupe CH₃, un groupe OH, un groupe NH₂, un groupe NHCOCH₃, un groupe COOH, un groupe CH₂SH et un groupe C(CH₃)₂SH ; lorsque la liaison entre les atomes de carbone C^{A} et C^{B} représente une liaison double R⁵ et R⁶ ou R^{6'} et R^{5'} sont absents ;
avec cette réserve que, lorsque Y est choisi parmi les radicaux divalents mentionnés sous c)-d), le groupe -ONO₂ est lié à un groupe -(CH₂)ₙ₁- ;
avec cette réserve que, lorsque s répondant à la formule (I) est égal à 1 et lorsque Z représente un groupe -(CO)-, le radical divalent Y n'a pas les significations reprises sous a), b) et d) ;
e)
un groupe 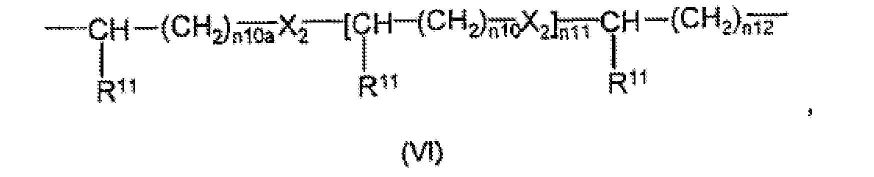 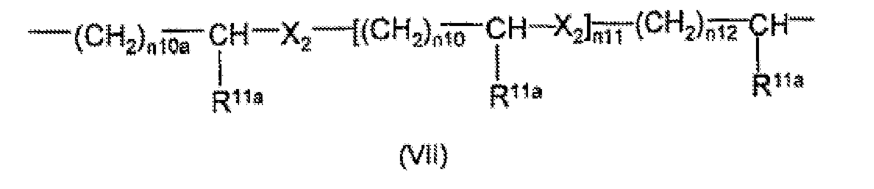 dans lequel X₂ représente un atome d'oxygène ou un atome de soufre ;
n10a, n10 et n12 représentent des entiers choisis indépendamment les uns des autres parmi des entiers de 0 à 20 ;
n11 représente un entier de 0 à 6 ;
R¹¹ représente un atome d'hydrogène, un groupe CH₃ ou un groupe n itrooxy ;
avec cette réserve que, lorsque, dans la formule (I) s est égal à 1, dans la formule (II) Z représente un groupe -(CO)-, dans la formule (VI) du radical divalent Y, n10a, n10, n12 sont égaux à 1, X ne peut pas représenter un atome d'oxygène ;
f)
un groupe 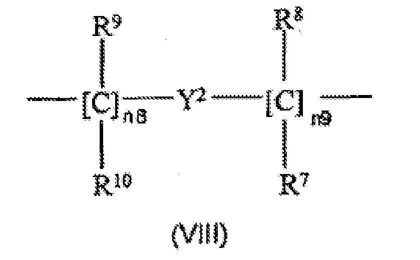
dans lequel:
n8 représente un entier de 0 à 10 ;
n9 représente un entier de 1 à 10 ;
R⁹, R¹⁰, R⁸, R⁷ sont identiques ou différents et représentent un atome d'hydrogène ou un groupe alkyle en C₁-C₄ à chaîne droite ou ramifiée ;
le groupe -ONO₂ est lié à : 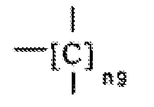 dans lequel n9 est tel que défini ci-dessus ;
Y² représente un noyau saturé hétérocyclique, insaturé ou aromatique à 5 ou 6 membres, contenant un ou plusieurs hétéroatomes choisis parmi un atome d'azote, un atome d'oxygène, un atome de soufre, et est choisi parmi le groupe constitué par :

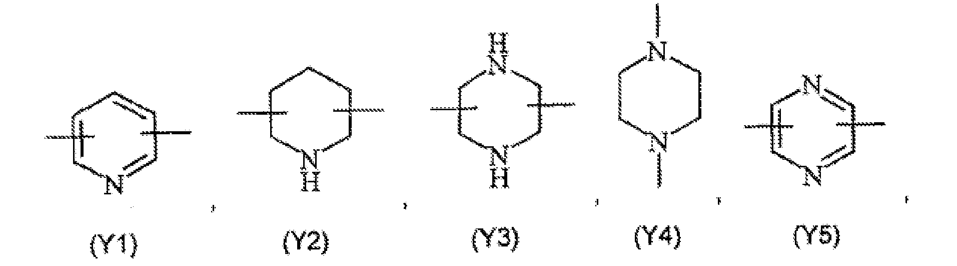

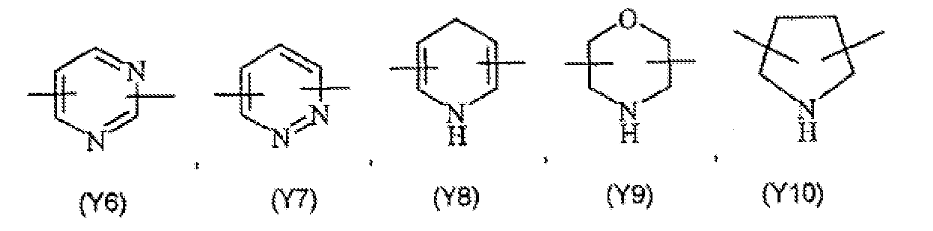

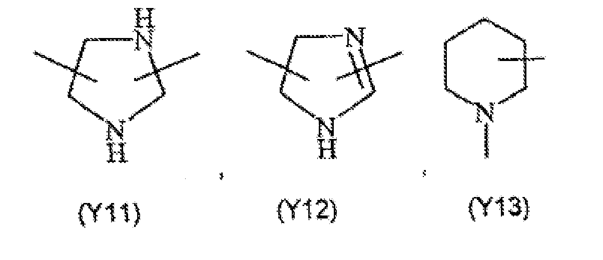

2. Composé, ainsi que ses énantiomères, ses diastéréo-isomères et ses sels pharmaceutiquement acceptables selon la revendication 1, dans lequel
s est égal à 1 et Z₁ représente un atome d'hydrogène.

3. Composé, ainsi que ses énantiomères, ses diastéréo-isomères et ses sels pharmaceutiquement acceptables selon la revendication 2, dans lequel Z représente un groupe -C(O)-.

4. Composé, ainsi que ses énantiomères, ses diastéréo-isomères et ses sels pharmaceutiquement acceptables selon la revendication 3, dans lequel
Y représente un groupe 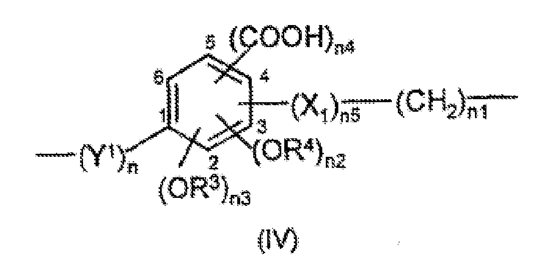 dans lequel
n, n2, n3, n4 et n5 sont égaux à 0 ;
n1 représente un entier égal à 1.

5. Composé, ainsi que ses énantiomères, ses diastéréo-isomères et ses sels pharmaceutiquement acceptables selon la revendication 3, dans lequel
Y représente un groupe 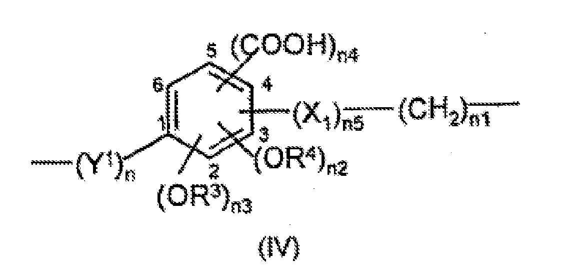 dans lequel
n, n2, n5 sont égaux à 1 ;
n3 et n4 sont égaux à 0 ;
n1 représente un entier de 1 à 10 ;
Y¹ représente un groupe -(CH₂)ₙₐ-CH=CH- dans lequel na est égal à 0 ;
X₁ représente un groupe -WC(O)- dans lequel W représente un atome d'oxygène et X₁ est lié au noyau phényle via l'atome de carbone [C]₄;
R⁴ représente un groupe CH₃ et le groupe (OR⁴) est lié au noyau phényle via l'atome de carbone [C]₃.

6. Composé, ainsi que ses énantiomères, ses diastéréo-isomères et ses sels pharmaceutiquement acceptables selon la revendication 3, dans lequel
Y représente un groupe 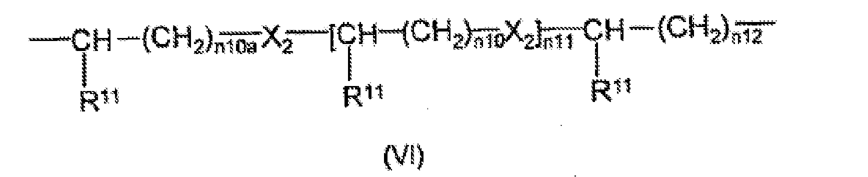 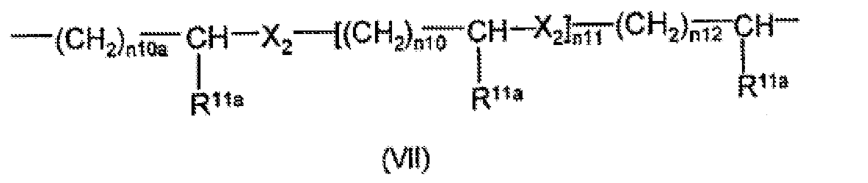 dans lequel
X₂ représente un atome d'oxygène ou un atome de soufre ;
n10a, n10 et n12 représentent des entiers choisis, indépendamment les uns des autres, parmi des entiers de 2 à 20 ;
n11 représente un entier de 0 à 6 ;
R¹¹ représente un atome d'hydrogène, un groupe CH₃ ou un groupe nitrooxy ;
R^{11a} représente un groupe CH₃ ou un groupe nitrooxy.

7. Composé, ainsi que ses énantiomères, ses diastéréo-isomères et ses sels pharmaceutiquement acceptables selon la revendication 2, dans lequel Z représente un groupe -C(O)O-.

8. Composé, ainsi que ses énantiomères, ses diastéréo-isomères et ses sels pharmaceutiquement acceptables selon la revendication 7, dans lequel
Y représente un groupe alkylène en C₁-C₂₀ à chaîne droite ou ramifiée substitué de manière facultative par un ou plusieurs des substituants choisis parmi le groupe constitué par un atome d'halogène, un groupe hydroxyle, un groupe -ONO₂ ou un groupe T, T représentant un groupe - OC(O)(alkyl en C₁-C₁₀)-ONO₂, un groupe -O(alkyl en C₁-C₁₀)-ONO₂.

9. Composé, ainsi que ses énantiomères, ses diastéréo-isomères et ses sels pharmaceutiquement acceptables selon la revendication 8, dans lequel
Y représente un groupe alkylène en C₁-C₁₀ à chaîne droite ou ramifiée.

10. Composé, ainsi que ses énantiomères, ses diastéréo-isomères et ses sels pharmaceutiquement acceptables selon la revendication 7, dans lequel
Y représente un groupe 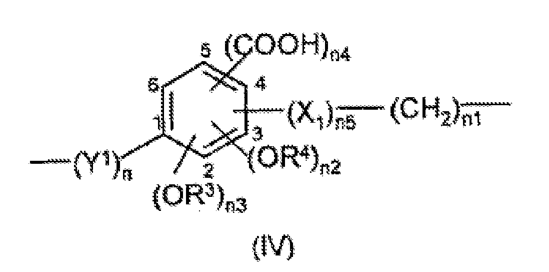 dans lequel
n représente un entier de 0 à 20 ;
n1 représente un entier de 1 à 20 ;
n2, n3, n4 et n5 représentent des entiers égaux ou différents les uns des autres, égaux à 0 ou 1 ;
R³ et R⁴ sont choisis, indépendamment l'un de l'autre, parmi un atome d'hydrogène ou un groupe CH₃ ;
Y¹ représente un groupe -CH₂- ou un groupe -(CH₂)ₙₐ-CH=CH- dans lequel na représente un entier de 0 à 20 ;
X₁ représente un groupe -WC(O)- ou un groupe -C(O)W- dans lequel W représente un atome d'oxygène, un atome de soufre ou un groupe NH.

11. Composé, ainsi que ses énantiomères, ses diastéréo-isomères et ses sels pharmaceutiquement acceptables selon la revendication 10, dans lequel
n2, n3, n4, n5 sont égaux à 0 ;
n1 est égal à 1 ;
n représente un entier de 0 à 10 ;
Y¹ représente un groupe CH₂.

12. Composé, ainsi que ses énantiomères, ses diastéréo-isomères et ses sels pharmaceutiquement acceptables selon la revendication 7, dans lequel
Y représente un groupe 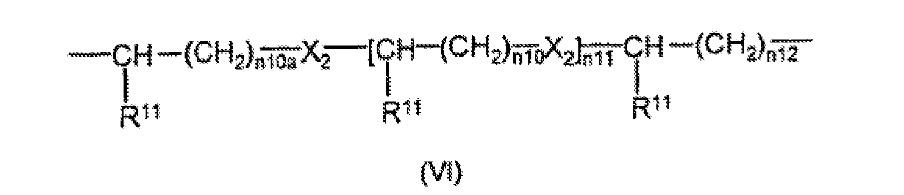 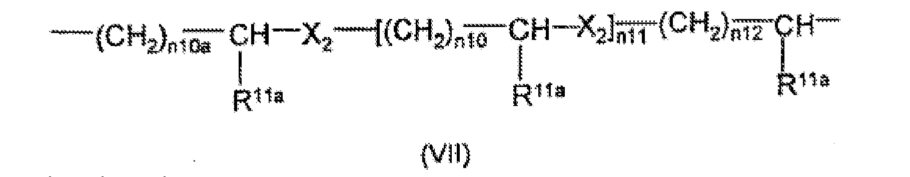 dans lequel
X₂ représente un atome d'oxygène ou un atome de soufre ;
n10a, n10 et n12 représentent des entiers choisis, indépendamment les uns des autres, parmi des entiers de 0 à 20 ;
n11 représente un entier de 0 à 6 ;
R¹¹ représente un atome d'hydrogène, un groupe CH₃ ou un groupe n itrooxy ;
R^{11a} représente un groupe CH₃ ou un groupe nitrooxy.

13. Composé, ainsi que ses énantiomères, ses diastéréo-isomères et ses sels pharmaceutiquement acceptables selon la revendication 12, dans lequel
Y représente un groupe 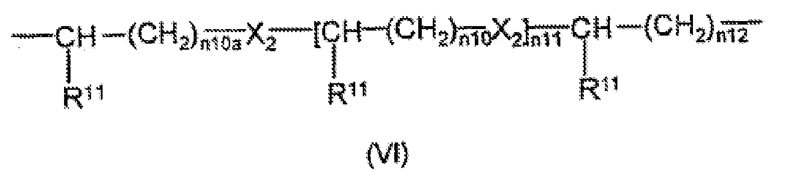 dans lequel
X₂ représente un atome d'oxygène ou un atome de soufre ;
n10a et n11 sont égaux à 0 ;
n12 est égal à 1 ; et
R¹¹ représente un atome d'hydrogène ;
le groupe -ONO₂- étant lié au groupe -(CH₂)ₙ₁₂-.

14. Composé, ainsi que ses énantiomères, ses diastéréo-isomères et ses sels pharmaceutiquement acceptables selon la revendication 7, dans lequel
Y représente un groupe 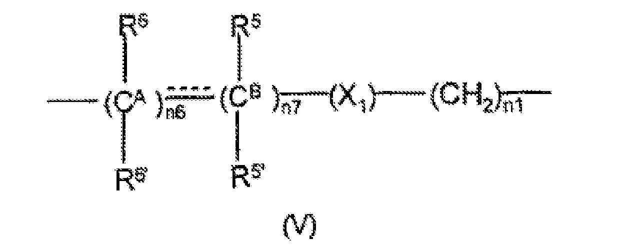 dans lequel
n1 représente un entier de 1 à 20 ;
X₁ représente un groupe -WC(O)- ou un groupe -C(O)W- dans lequel W représente un atome d'oxygène, un atome de soufre ou un groupe NH ;
n6 représente un entier de 1 à 20 ;
n7 représente un entier de 0 à 20 ;
R⁵ et R^{5'}, R⁶ et R^{6'} sont choisis, indépendamment l'un de l'autre, parmi le groupe constitué par un atome d'hydrogène, un groupe CH₃, un groupe OH, un groupe NH₂, un groupe NHCOCH₃, un groupe COOH, un groupe CH₂SH et un groupe C(CH₃)₂SH ; lorsque la liaison entre les atomes de carbone C^{A} et C^{B} représente une liaison double R⁵ et R⁶ ou R^{6'} et R^{5'} sont absents.

15. Composé, ainsi que ses énantiomères, ses diastéréo-isomères et ses sels pharmaceutiquement acceptables selon la revendication 2, dans lequel Z représente un groupe

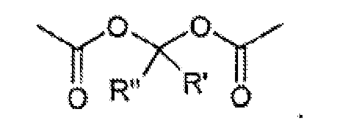

16. Composé, ainsi que ses énantiomères, ses diastéréo-isomères et ses sels pharmaceutiquement acceptables selon la revendication 15, dans lequel
Y représente un groupe alkylène en C₁-C₂₀ à chaîne droite ou ramifiée substitué de manière facultative par un ou plusieurs des substituants choisis parmi le groupe constitué par un atome d'halogène, un groupe hydroxyle, un groupe -ONO₂ ou un groupe T, T représentant un groupe - OC(O)(alkyl en C₁-C₁₀)-ONO₂, un groupe -O(alkyl en C₁-C₁₀)-ONO₂.

17. Composé, ainsi que ses énantiomères, ses diastéréo-isomères et ses sels pharmaceutiquement acceptables selon la revendication 16, dans lequel Y représente un groupe alkylène en C₁-C₁₀ à chaîne droite ou ramifiée.

18. Composé, ainsi que ses énantiomères, ses diastéréo-isomères et ses sels pharmaceutiquement acceptables selon la revendication 15, dans lequel
Y représente un groupe 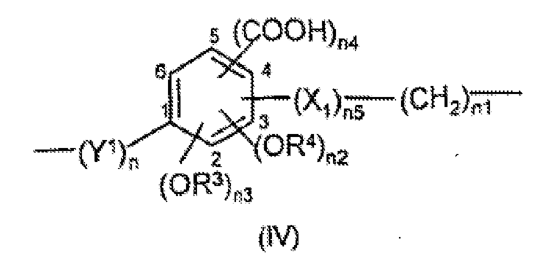 dans lequel
n représente un entier de 0 à 20 ;
n1 représente un entier de 1 à 20 ;
n2, n3, n4 et n5 représentent des entiers égaux ou différents les uns des autres, égaux à 0 ou 1 ;
R³ et R⁴ sont choisis, de manière indépendante, parmi un atome d'hydrogène ou un groupe CH₃ ;
Y¹ représente un groupe -CH₂- ou -(CH₂)ₙₐ-CH=CH- dans lequel na représente un entier de 0 à 20 ;
X₁ représente un groupe -WC(O)- ou un groupe -C(O)W- dans lequel W représente un atome d'oxygène, un atome de soufre ou un groupe NH.

19. Composé, ainsi que ses énantiomères, ses diastéréo-isomères et ses sels pharmaceutiquement acceptables selon la revendication 18, dans lequel
n2, n3, n4 et n5 sont égaux à 0 ;
n1 est égal à 1 ;
n représente un entier de 0 à 10 ;
Y¹ représente un groupe CH₂.

20. Composé, ainsi que ses énantiomères, ses diastéréo-isomères et ses sels pharmaceutiquement acceptables selon la revendication 1, dans lequel Z et Z₁ représentent un groupe -C(O)-.

21. Composé, ainsi que ses énantiomères, ses diastéréo-isomères et ses sels pharmaceutiquement acceptables selon la revendication 20, dans lequel
Y représente un groupe alkylène en C₁-C₂₀ à chaîne droite ou ramifiée substitué de manière facultative par un ou plusieurs des substituants choisis parmi le groupe constitué par un atome d'halogène, un groupe hydroxyle, un groupe -ONO₂ ou un groupe T, T représentant un groupe - OC(O)(alkyl en C₁-C₁₀)-ONO₂, un groupe -O(alkyl en C₁-C₁₀)-ONO₂.

22. Composé, ainsi que ses énantiomères, ses diastéréo-isomères et ses sels pharmaceutiquement acceptables selon la revendication 21, dans lequel Y représente un groupe alkylène en C₁-C₁₀ à chaîne droite ou ramifiée.

23. Composé, ainsi que ses énantiomères, ses diastéréo-isomères et ses sels pharmaceutiquement acceptables selon la revendication 20, dans lequel
Y représente un groupe 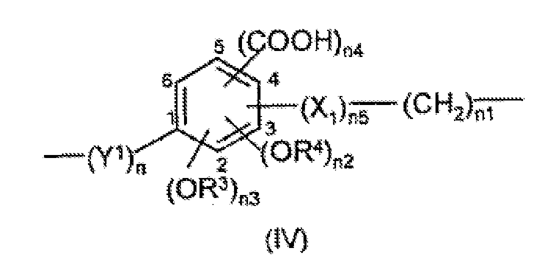 dans lequel
n représente un entier de 0 à 20 ;
n1 représente un entier de 1 à 20 ;
n2, n3, n4 et n5 représentent des entiers égaux ou différents les uns des autres, égaux à 0 ou 1 ;
R³ et R⁴ sont choisis, indépendamment l'un de l'autre, parmi un atome d'hydrogène ou un groupe CH₃ ;
Y¹ représente un groupe -CH₂- ou un groupe -(CH₂)ₙₐ-CH=CH- dans lequel na représente un entier de 0 à 20 ;
X₁ représente un groupe -WC(O)- ou un groupe -C(O)W- dans lequel W représente un atome d'oxygène, un atome de soufre ou un groupe NH.

24. Composé, ainsi que ses énantiomères, ses diastéréo-isomères et ses sels pharmaceutiquement acceptables selon la revendication 23, dans lequel
n2, n3, n4, n5 sont égaux à 0 ;
n1 est égal à 1 ;
n représente un entier de 0 à 10 ;
Y¹ représente un groupe CH₂.

25. Composé, ainsi que ses énantiomères, ses diastéréo-isomères et ses sels pharmaceutiquement acceptables selon la revendication 23, dans lequel
n, n2, n5 sont égaux à 1 ;
n3 et n4 sont égaux à 0 ;
n1 représente un entier de 1 à 10 ;
Y¹ représente un groupe -(CH₂)ₙₐ-CH=CH- dans lequel na est égal à 0 ;
X₁ représente un groupe -WC(O)- dans lequel W représente un atome d'oxygène et X₁ est lié au noyau phényle via l'atome de carbone [C]₄, R⁴ représente un groupe CH₃ et le groupe (OR⁴) est lié au noyau phényle via l'atome de carbone [C]₃.

26. Composé, ainsi que ses énantiomères, ses diastéréo-isomères et ses sels pharmaceutiquement acceptables selon la revendication 20, dans lequel
Y représente un groupe 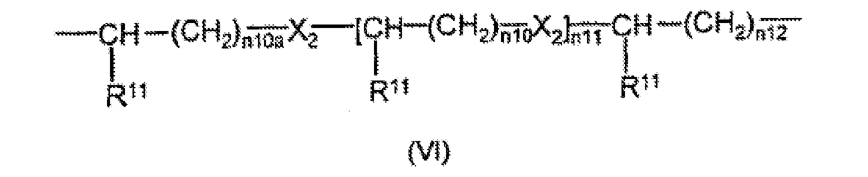 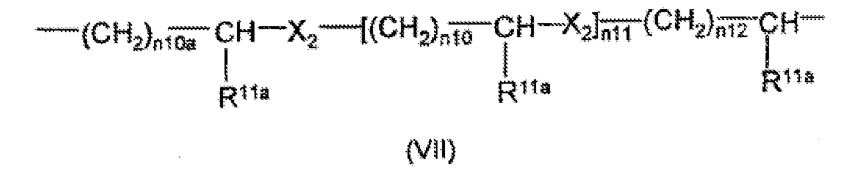 dans lequel
X₂ représente un atome d'oxygène ou un atome de soufre ;
n10a, n10 et n12 représentent des entiers choisis, indépendamment les uns des autres, parmi des entiers de 0 à 20 ;
n11 représente un entier de 0 à 6 ;
R¹¹ représente un atome d'hydrogène, un groupe CH₃ ou un groupe n itrooxy ;
R^{11a} représente un groupe CH₃ ou un groupe nitrooxy.

27. Composé, ainsi que ses énantiomères, ses diastéréo-isomères et ses sels pharmaceutiquement acceptables selon la revendication 26, dans lequel
Y représente un groupe 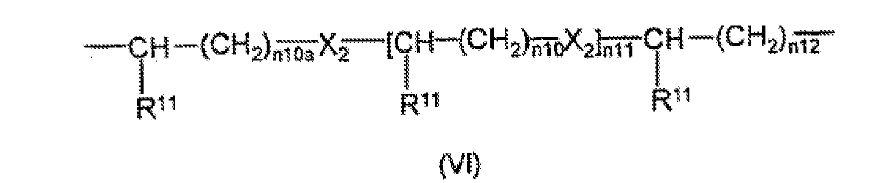 dans lequel
X₂ représente un atome d'oxygène ou un atome de soufre ;
n10a et n11 sont égaux à 0 ;
n12 est égal à 1 ;
R¹¹ représente un atome d'hydrogène ;
le groupe -ONO₂- étant lié au groupe -(CH₂)ₙ₁₂-.

28. Composé, ainsi que ses énantiomères, ses diastéréo-isomères et ses sels pharmaceutiquement acceptables selon la revendication 20, dans lequel
Y représente un groupe 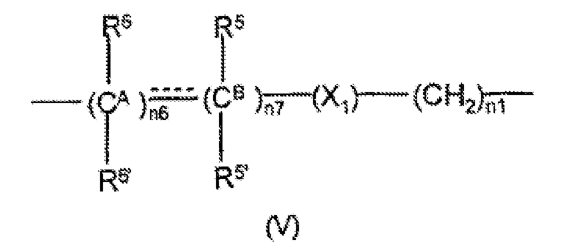 dans lequel
n1 représente un entier de 1 à 20 ;
X₁ représente un groupe -WC(O)- ou un groupe -C(O)W- dans lequel W représente un atome d'oxygène, un atome de soufre ou un groupe NH ;
n6 représente un entier de 1 à 20 ;
n7 représente un entier de 0 à 20 ;
R⁵ et R^{5'}, R⁶ et R^{6'} sont choisis, indépendamment l'un de l'autre, parmi le groupe constitué par un atome d'hydrogène, un groupe CH₃, un groupe OH, un groupe NH₂, un groupe NHCOCH₃, un groupe COOH, un groupe CH₂SH et un groupe C(CH₃)₂SH ; lorsque la liaison entre les atomes de carbone C^{A} et C^{B} représente une liaison double R⁵ et R⁶ ou R^{6'} et R^{5'} sont absents.

29. Composé, ainsi que ses énantiomères, ses diastéréo-isomères et ses sels pharmaceutiquement acceptables selon la revendication 28, dans lequel
n1 représente un entier de 1 à 10 ;
n6 et n7 sont égaux à 1 ;
X₁ représente un groupe -WC(O) dans lequel W représente un atome de soufre ;
R⁵, R^{5'} et R^{6'} représentent un atome d'hydrogène ;
R⁶ représente un groupe NHCOCH₃ ;
avec cette réserve que le groupe -ONO₂ est lié au groupe -(CH2)ₙ₁-.

30. Composé, ainsi que ses énantiomères, ses diastéréo-isomères et ses sels pharmaceutiquement acceptables selon la revendication 1, dans lequel s est égal à 1, Z représente un atome d'hydrogène et Z₁ représente un groupe -C(O)-.

31. Composé, ainsi que ses énantiomères, ses diastéréo-isomères et ses sels pharmaceutiquement acceptables selon la revendication 30, dans lequel
Y représente un groupe alkylène en C₁-C₂₀ à chaîne droite ou ramifiée substitué de manière facultative par un ou plusieurs des substituants choisis parmi le groupe constitué par un atome d'halogène, un groupe hydroxyle, un groupe -ONO₂ ou un groupe T, T représentant un groupe - OC(O)(alkyl en C₁-C₁₀)-ONO₂, un groupe -O(alkyl en C₁-C₁₀)-ONO₂.

32. Composé, ainsi que ses énantiomères, ses diastéréo-isomères et ses sels pharmaceutiquement acceptables selon la revendication 31, dans lequel Y représente un groupe alkylène en C₁-C₁₀ à chaîne droite ou ramifiée.

33. Composé, ainsi que ses énantiomères, ses diastéréo-isomères et ses sels pharmaceutiquement acceptables selon la revendication 30, dans lequel
Y représente un groupe 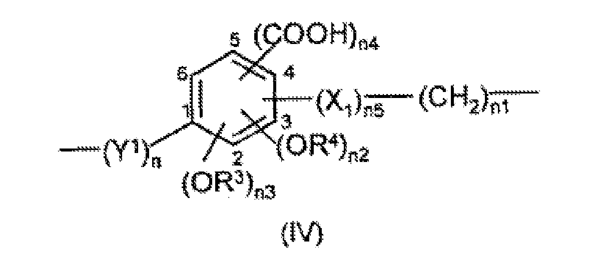 dans lequel
n représente un entier de 0 à 20 ;
n1 représente un entier de 1 à 20 ;
n2, n3, n4 et n5 représentent des entiers égaux ou différents les uns des autres, égaux à 0 ou 1 ;
R³ et R⁴ sont choisis, indépendamment l'un de l'autre, parmi un atome d'hydrogène ou un groupe CH₃ ;
Y¹ représente un groupe -CH₂- ou un groupe -(CH₂)ₙₐ-CH=CH- dans lequel na représente un entier de 0 à 20 ;
X₁ représente un groupe -WC(O)-ou un groupe -C(O)W- dans lequel W représente un atome d'oxygène, un atome de soufre ou un groupe NH.

34. Composé, ainsi que ses énantiomères, ses diastéréo-isomères et ses sels pharmaceutiquement acceptables selon la revendication 33, dans lequel
n2, n3, n4, n5 sont égaux à 0 ;
n1 est égal à 1 ;
n représente un entier de 0 à 10 ;
Y¹ représente un groupe CH₂.

35. Composé, ainsi que ses énantiomères, ses diastéréo-isomères et ses sels pharmaceutiquement acceptables selon la revendication 33, dans lequel
n, n2, n5 sont égaux à 1 ;
n3 et n4 sont égaux à 0 ;
n1 représente un entier de 1 à 10 ;
Y¹ représente un groupe -(CH₂)ₙₐ-CH=CH- dans lequel na est égal à 0 ;
X₁ représente un groupe -WC(O)- dans lequel W représente un atome d'oxygène et X₁ est lié au noyau phényle via l'atome de carbone [C]₄, R⁴ représente un groupe CH₃ et le groupe (OR⁴) est lié au noyau phényle via l'atome de carbone [C]₃.

36. Composé, ainsi que ses énantiomères, ses diastéréo-isomères et ses sels pharmaceutiquement acceptables selon la revendication 30, dans lequel
Y représente un groupe 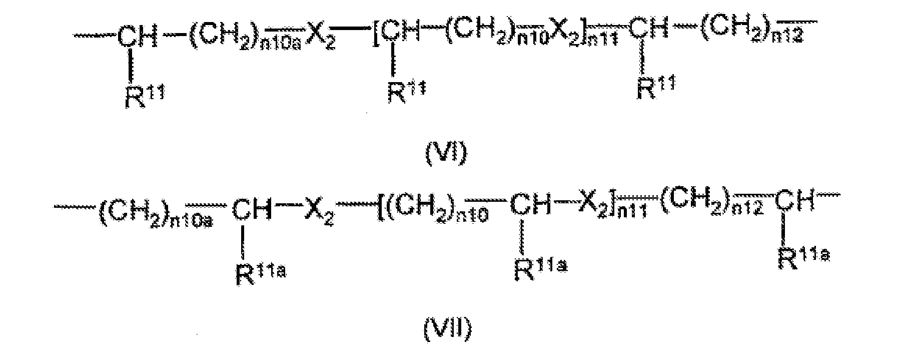 dans lequel
X₂ représente un atome d'oxygène ou un atome de soufre ;
n10a, n10 et n12 représentent des entiers choisis, indépendamment les uns des autres, parmi des entiers de 0 à 20 ;
n11 représente un entier de 0 à 6 ;
R¹¹ représente un atome d'hydrogène, un groupe CH₃ ou un groupe n itrooxy ;
R^{11a} représente un groupe CH₃ ou un groupe nitrooxy.

37. Composé, ainsi que ses énantiomères, ses diastéréo-isomères et ses sels pharmaceutiquement acceptables selon la revendication 36, dans lequel
Y représente un groupe 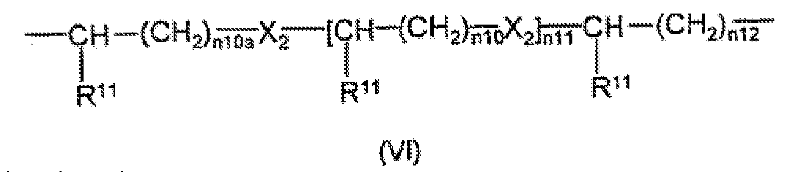 dans lequel
X₂ représente un atome d'oxygène ou un atome de soufre ;
n10a et n11 sont égaux à 0 ;
n12 est égal à 1 ;
R¹¹ représente un atome d'hydrogène ;
le groupe -ONO₂- étant lié au groupe -(CH₂)ₙ₁₂-.

38. Composé, ainsi que ses énantiomères, ses diastéréo-isomères et ses sels pharmaceutiquement acceptables selon la revendication 30, dans lequel
Y représente un groupe 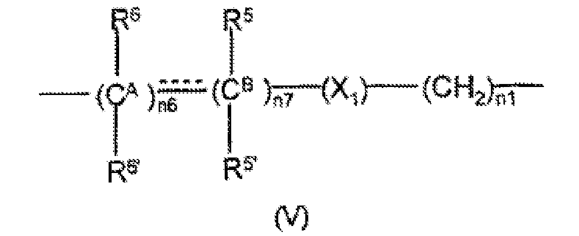 dans lequel
n1 représente un entier de 1 à 20 ;
X₁ représente un groupe -WC(O)- ou un groupe -C(O)W- dans lequel W représente un atome d'oxygène, un atome de soufre ou un groupe NH ;
n6 représente un entier de 1 à 20 ;
n7 représente un entier de 0 à 20 ;
R⁵ et R^{5'}, R⁶ et R^{6'} sont choisis, indépendamment l'un de l'autre, parmi le groupe constitué par un atome d'hydrogène, un groupe CH₃, un groupe OH, un groupe NH₂, un groupe NHCOCH₃, un groupe COOH, un groupe
CH₂SH et un groupe C(CH₃)₂SH ;
lorsque la liaison entre les atomes de carbone C^{A} et C^{B} représente une liaison double R⁵ et R⁶ ou R^{6'} et R^{5'} sont absents.

39. Composé, ainsi que ses énantiomères, ses diastéréo-isomères et ses
sels pharmaceutiquement acceptables selon la revendication 38, dans lequel
n1 représente un entier de 1 à 10 ;
n6 et n7 sont égaux à 1 ;
X₁ représente un groupe -WC(O)- dans lequel W représente un atome de soufre ;
R⁵, R^{5'} et R^{6'} représentent un atome d'hydrogène ;
R⁶ représente un groupe NHCOCH₃ ;
avec cette réserve que le groupe -ONO₂ est lié au groupe -(CH2)ₙ₁-.

40. Composés, ainsi que leurs énantiomères, leurs diastéréo-isomères et leurs sels pharmaceutiquement acceptables selon les revendications 3 et 4, dans lesquels les composés sont :

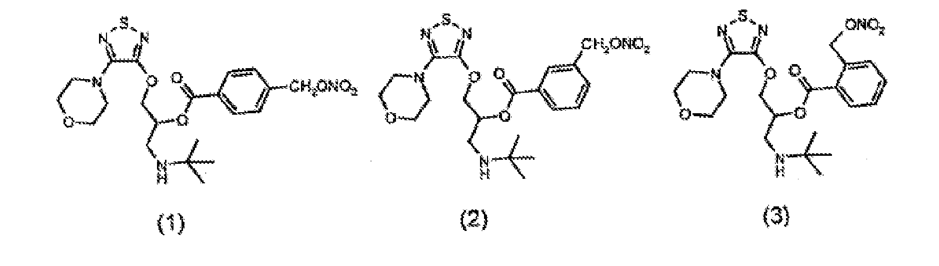

41. Composés, ainsi que leurs énantiomères, leurs diastéréo-isomères et leurs sels pharmaceutiquement acceptables selon les revendications 20 et 24, dans lesquels les composés sont :

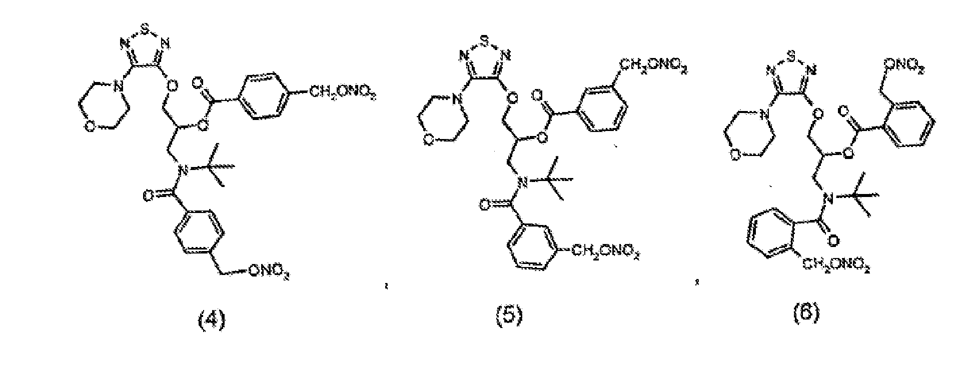

42. Composés, ainsi que leurs énantiomères, leurs diastéréo-isomères et leurs sels pharmaceutiquement acceptables selon les revendications 30 et 34, dans lesquels les composés sont :

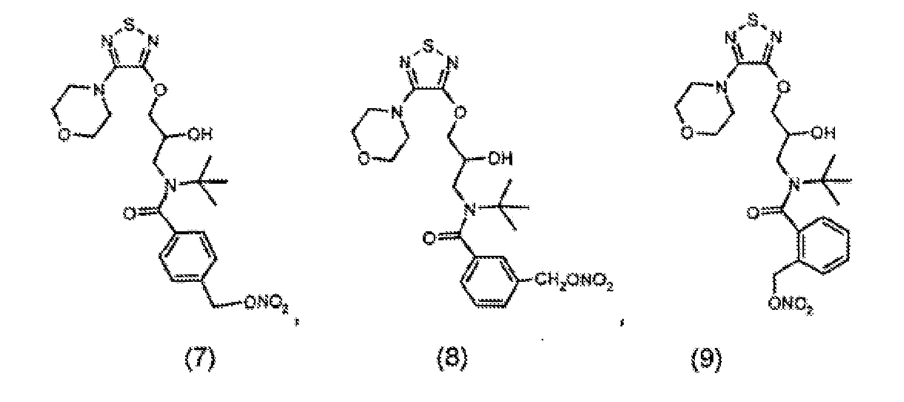

43. Composé, ainsi que ses énantiomères, ses diastéréo-isomères et ses sels pharmaceutiquement acceptables selon les revendications 1 et 4, à savoir le sel (S)-1-[(1,1-diméthyléthyl)amino]-3-[[4-(4-morpholinyl)-1,2,5-thiadiazol-3-yl]oxy]-2-propanoate maléate de l'acide 4-(nitrooxyméthyl)-benzoïque.

44. Composé, ainsi que ses énantiomères, ses diastéréo-isomères et ses sels pharmaceutiquement acceptables selon les revendications 1 et 24, à savoir le sel (S)-1-[(1,1-diméthyléthyl)[(4-nitrooxyméthyl)benzoyl]amino]-3-[[4-(4-morpholinyl)-1,2,5-thiadiazol-3-yl]oxy]-2-propanoate de l'acide 4-(nitrooxyméthyl)benzoïque.

45. Composé, ainsi que ses énantiomères, ses diastéréo-isomères et ses sels pharmaceutiquement acceptables selon les revendications 1 et 34, à savoir le sel (S)-1-[(1,1-diméthyléthyl)[(4-nitrooxyméthyl)benzoyl]amino]-3-[[4-(4-morpholinyl)-1,2,5-thiadiazol-3-yl]oxy]-2-propanol.

46. Composé répondant à la formule (I), et/ou ses énantiomères, ses diastéréo-isomères et ses sels pharmaceutiquement acceptables, tel que définis dans l'une quelconque des revendications 1 à 45, à utiliser comme médicament.

47. Utilisation d'un composé répondant à la formule (I), et/ou ses énantiomères, ses diastéréo-isomères et ses sels pharmaceutiquement acceptables, tels que définis dans l'une quelconque des revendications 1 à 45, pour préparer un médicament qui peut être utilisé dans le traitement ou dans la prophylaxie de l'hypertension, des maladies cardiovasculaires et des maladies vasculaires.

48. Utilisation d'un composé répondant à la formule (I), et/ou ses énantiomères, ses diastéréo-isomères et ses sels pharmaceutiquement acceptables, tels que définis dans l'une quelconque des revendications 1 à 45, pour préparer un médicament qui peut être utilisé dans le traitement du glaucome et de la pression intraoculaire élevée.

49. Composition pharmaceutique comprenant un composé répondant à la formule (I), et/ou ses énantiomères, ses diastéréo-isomères et ses sels pharmaceutiquement acceptables, tels que définis dans l'une quelconque des revendications 1 à 45, et un support pharmaceutiquement acceptable.
